(19) Europäisches Patentamt / European Patent Office / Office européen des brevets

(11) **EP 3 824 469 B1**

(12) **EUROPEAN PATENT SPECIFICATION**

(45) Date of publication and mention of the grant of the patent:
**19.02.2025 Bulletin 2025/08**

(21) Application number: **19746143.7**

(22) Date of filing: **29.05.2019**

(51) International Patent Classification (IPC):
**G16B 5/30** (2019.01)

(52) Cooperative Patent Classification (CPC):
**G16B 5/30**

(86) International application number:
**PCT/IB2019/054431**

(87) International publication number:
**WO 2020/016669 (23.01.2020 Gazette 2020/04)**

(54) **METHOD FOR CALCULATING KINETIC PARAMETERS OF A REACTION NETWORK**

VERFAHREN ZUR BERECHNUNG VON KINETISCHEN PARAMETERN EINES REAKTIONSNETZWERKS

PROCÉDÉ DE CALCUL DE PARAMÈTRES CINÉTIQUES D'UN RÉSEAU DE RÉACTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.07.2018 CH 8872018**

(43) Date of publication of application:
**26.05.2021 Bulletin 2021/21**

(73) Proprietor: **Creoptix AG**
**8820 Wädenswil (CH)**

(72) Inventors:
• **FIEVET, Lucas**
**8041 Zürich (CH)**
• **COTTIER, Kaspar**
**8820 Wädenswil (CH)**

(74) Representative: **P&TS SA (AG, Ltd.)**
**Avenue J.-J. Rousseau 4**
**P.O. Box 2848**
**2001 Neuchâtel (CH)**

(56) References cited:
• **KAMALAKANTA ROUTRAY ET AL: "Kinetic parameter estimation for a multiresponse nonlinear reaction model", AICHE JOURNAL, JOHN WILEY & SONS, INC, US, vol. 51, no. 6, 21 April 2005 (2005-04-21), pages 1733 - 1746, XP071002606, ISSN: 0001-1541, DOI: 10.1002/AIC.10446**
• **SARAH E HOLTE: "A Consistent Direct Method for Estimating Parameters in Ordinary Differential Equations Models", 12 February 2018 (2018-02-12), pages 1 - 34, XP055621599, Retrieved from the Internet <URL:https://arxiv.org/pdf/1601.04736.pdf> [retrieved on 20190912]**
• **ITAI DATTNER ET AL: "Optimal rate of direct estimators in systems of ordinary differential equations linear in functions of the parameters", ELECTRONIC JOURNAL OF STATISTICS, vol. 9, no. 2, 1 January 2015 (2015-01-01), pages 1939 - 1973, XP055621605, DOI: 10.1214/15-EJS1053**

**Description**

Field of the invention

The present invention concerns methods for calculating kinetic parameters of a reaction network which involves providing an intermediate objective function which comprises a linearized intermediate discrepancy function, wherein all hidden states have been eliminated, and minimizing the intermediate objective function, using a direct estimation method.

Description of related art

A chemical or biochemical reaction network describes the reactions, in general reversible, occurring between a set of reactants to form a set of products, wherein a reactant is a substance that takes part in and undergoes change during a reaction and a product is a substance that is the result of a reaction between one or more reactants. A reactant can simultaneously be a product and a product can simultaneously be a reactant in a reaction network.

The state of a reaction network is given by the set of concentrations of all reactants and products in the reaction network, often represented by a single concentration vector.

Each reaction in a reaction network can be described by a differential equation in the concentrations of the involved reactants and products, said differential equation comprising kinetic parameters that determine the reaction velocities among the reactants. The time evolution of the state of a reaction network is determined by the set of differential equations resulting from all reactions in the network. It should be understood that different reaction networks are described by different set of differential equations, comprising different kinetic parameters.

Determining the kinetic parameters of a reaction network is of interest in chemical and biochemical applications. For example, in drug development the kinetic parameters of a reaction network comprising as reactants a drug and a drug target determine the suitability of the drug. In bioprocessing, the kinetic parameters of a reaction network are useful to optimize a bioreactor so as obtain a desired product with a minimal amount of the reactant substances and in a minimal amount of time.

The kinetic parameters of a reaction network are determined from a sequence over time of measurements of the reaction network state, or a part of the reaction network state. Wherein a measurement of a part of the reaction network state consists of a set of numerical values for one or more concentrations of the reactants and products in the reaction network state at a given time. Hereafter, a signal is a sequence over time of measurements of a part of the reaction network state, or of measurements of the reaction network state.

It is intuitively understood that a signal is acquired, or measured, by performing an acquisition method using a physical device (such as biochemical sensors). An acquisition method typically has physical limitations and the acquired signal contains noise and artefacts resulting from these physical limitations of the acquisition method.

A requirement on the signal is that the time interval between two consecutive measurements in a signal is smaller than the time scale of the reaction network for which the kinetic parameters are to be determined. The time scale of the reaction network is defined as the time the state of a reaction network needs to reach the equilibrium state, or the time of the shortest oscillation for the state of a periodic reaction network. The equilibrium state is the state in which the reaction network no longer changes over time and remains in the equilibrium state. The state of a periodic reaction network never reaches equilibrium but oscillates over time with a repeating pattern.

Acquisition methods performed to acquire a molecular interaction signal, molecular interaction which is described by a respective reaction network, rely on biochemical sensors. These biochemical sensors can be split into two categories; namely label-free sensors and sensors which operate using a marker. These sensors are generally known in the field of life sciences and are mainly used for the characterization of interactions of biologic or biochemical molecules. These characterizations commonly involve bringing two or more reactants, such as different types of sample molecules, into physical contact with each other for a set period of time and recording the signal for said period of time.

Acquisition methods using sensors which operate using a marker involve chemically attaching a marker, typically a fluorescent, absorbing or radioactive molecule, to a reactant or product which is to be detected. Here, the concentration of a state in a reaction network is determined indirectly by measuring the concentration of the marker attached to the reactant or product.

US 8,325,347 B2 describes an acquisition method which uses a label-free optical sensor to acquire a molecular interaction signal; the method is based on the waveguide interferometry where the local refractive index near the sensor surface is probed by an evanescent wave of a waveguide mode and read out by interferometry.

Acquisition methods which use label-free sensors include, but are not limited to, optical methods based on surface plasmon resonance (SPR) or waveguides, or methods based on surface- acoustic waves (SAW), thermal methods, or electro-chemical methods. Optical methods are based on the principle that biochemical molecules exhibit a different refractive index than an aqueous solution. Refractive index changes near the sensor surface result from the addition or subtraction of molecules to the surfaces due to the interaction of molecules with either the sensor surface itself or another molecule attached to the surface. Using a resonant element- in case of SPR a metal layer supporting surface plasmons, or in case of waveguide sensors an optical waveguide supporting optical waveguide modes - the local refractive index changes can be then probed using an appropriate illumination and detection scheme, and the changes recorded in real time in order to measure the molecular reaction event. In this context, these changes correspond to the signal. Surface-Acoustic Wave and electro- chemical methods operate in a similar way, except that not the refractive index differences are physically measured, but rather mass or permittivity differences.

An exemplary type of a known label-free optical sensor, which can be used in an acquisition method for molecular interactions, is a surface plasmon resonance-based biosensor (or SPR-based biosensor). SPR-based biosensors utilize a SPR based mass-sensing technique to provide "real-time" signals between a surface bound ligand and an analyte of interest. The SPR-based biosensor produces as output signal a sequence in time of density measurements in pg/mm2, density measurements which are proportional to the concentration of the product on the sensor surface.

In the abovementioned applications, and potential further applications, not all states in a reaction network can be measured. Hence, the reaction network state is split into two components namely the 'hidden state' and the 'observed state'. The 'hidden state' is a set of reactant and product concentrations in the reaction network that are cannot be measured due to physical limitations; and the 'observed state', is a set of reactant and product concentrations in the reaction network that can be measured.

A 'model signal' is the expected signal as determined by the differential equations of a reaction network model.

Typically, the observed state is not observed directly but observed indirectly by a physically observable proxy state. The measured values over time for the physically observable proxy state are hereafter designated by observed signal. For example, the observed signal acquired by performing an acquisition method using a label-free biochemical sensor typically corresponds to a refractive index signal that is in (quasi-)linear relationship to the product concentration.

The observed signal is typically not equal to the model signal due to noise arising from the physical limitations of sensor(s) used in the acquisition method. The noise can be any combination of systematic deviations and stochastic deviations of the observed signal from the model signal.

A 'discrepancy function' is a mathematical expression for the discrepancies over time between an observed signal and a model signal or for the discrepancies over time of an observed signal, or its integral or derivative, with respect to a set of differential equations.

An 'objective function' is a function that summarizes a discrepancy function over time. The said objective function comprises at least a discrepancy function as parameter, where said discrepancy function comprises kinetic parameters. The objective function can further comprise one or more additional parameters. The values of the kinetic parameters that minimize said objective function are the values that best explain an observed signal in terms of a reaction network.

The procedure of finding the minima of the objective function is called minimization, where said minimization is performed using a mathematical optimization method.

Any minimization problem of an objective function is equivalent to the maximization of the opposite of the same objective function, mathematically expressed as

$$\operatorname{argmin}_{\vec{x}} \left( f_{objective}(\vec{x}) \right) = \operatorname{argmax}_{\vec{x}} \left( -f_{objective}(\vec{x}) \right). \qquad (1.)$$

In this application the term minimization will be used to refer to minimizing the discrepancies of an observed signal with respect to a reaction network model; however, it should be understood that every minimization step mentioned in this application could be replaced with a mathematically equivalent maximization step (since the minimization can always be rewritten as a mathematically equivalent maximization).

State of the art computation of kinetic parameters from an observed signal uses iterative fitting methods to minimize an objective function (Jesudason, 2011; Myszka, He, Dembo, Morton, & Goldstein, 1998; Voit, Martens, & Omholt, 2015). For example, (Myszka & Morton, 1998) use the Levenberg-Marquardt nonlinear minimization algorithm, and (Canedo & González-Hernández, 2011) use a second order gradient optimization algorithm. When the underlying reaction network model is not analytically solvable (e.g. mass transport model) an additional numerical integration scheme is needed to solve the differential equation(s). The repeated solving of the reaction network model, including necessary numerical integrations, is computationally intensive and delays the workflow of evaluating an observed signal. In particular, the workflow becomes inconveniently long when repeated evaluation is needed to tune the fit. Examples of parameters to tune are starting times and end times determining the observed signal range used for the computation. Further on, computing the estimation errors of the obtained values for the kinetic parameters is a complex problem (Johnson, Simpson, & Blom, 2009), which needs repeated fitting and becomes unfeasible on a single processor.

The simplest reaction network is the Langmuir model with the reversible reaction

$$A + B \rightleftharpoons AB, \qquad (2.)$$

where an analyte A and an immobilized ligand B reach equilibrium with the product AB. The reactants A and B associate to form the product AB, while simultaneously the reactant AB dissociates to form the products A and B. Hence, A, B and AB are simultaneously reactants and products.

Mathematically, the reaction network is described by the differential equations

$$\frac{d[AB]}{dt} = k_a[A][B] - k_d[AB] \qquad (a)$$

$$\frac{d[B]}{dt} = -k_a[A][B] + k_d[AB], \qquad (b) \qquad\qquad (3.)$$

where [A], [B] and [AB] are the concentrations of A, B and AB. The velocity of [A] and [B] associating to form the product [AB] is determined by the kinetic parameter $k_a$, and the velocity of [AB] dissociating to form the products [A] and [B] is determined by the kinetic parameter $k_d$.

An acquisition method is then performed to obtain an observed signal which, indirectly, represents the product concentration [AB]. When performing an acquisition method using a label-free sensor, the product concentration [AB] is observed indirectly via a refraction index signal. Importantly the analyte concentration [A] is held constant when performing the mentioned acquisition (the analyte concentration [A] is held constant for the duration of the refraction index signal). Hence, [A] and [AB] are herein observed states. In contrast, the ligand concentration [B] cannot be observed and is herein a hidden state. A sequence of different analyte concentrations results in a sequence of signals. In this example a label-free acquisition method is performed.

Despite the hidden state, the kinetic parameters are entirely determined by the two observed states. To eliminate the hidden state [B], the differential equations (2.a) and (2.b) are added and their sum is then integrated to obtain

$$\frac{d[AB]}{dt} + \frac{d[B]}{dt} = 0 \quad \Rightarrow \quad [B] = R_{max} - [AB], \qquad\qquad (4.)$$

where the integration introduces the 'integration constant' $R_{max}$ (or equivalently 'constant of integration'). This 'integration constant' is an additional non-kinetic parameter that needs to be determined.

Substituting the result of Equation (4.) in Equation (3.a) yields the differential equation

$$\frac{d[AB]}{dt} = k_a[A](R - [AB]) - k_d[AB], \qquad\qquad (5.)$$

which is a differential equation in the observed product concentration *[AB]* only.

During the association phase, the analyte has a constant concentration [A] = C, and the solution to the differential equation (5.) is given by

$$R(t) = [AB] = \frac{k_a C R_{max}}{k_a C + k_d}(1 - \exp(-(k_a C + k_d)t)), \qquad\qquad (6.)$$

where $R(t)$ is the model signal of the Langmuir model during the association phase.

During the dissociation phase, the analyte concentration is [A] = 0, and the solution of the differential equation is given by

$$R(t) = [AB] = R_0 \exp(-k_d t), \qquad\qquad (7.)$$

where $R(t)$ is the model signal of the Langmuir model during the dissociation phase, and $R_0$ is the model signal at the end of the association phase.

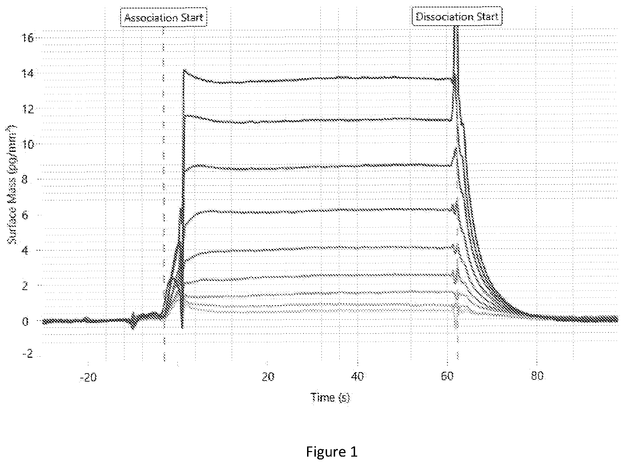

Figure 1 show baseline observed signals, association observed signals and dissociation observed signals for the Langmuir model at three different analyte concentrations and a fixed initial ligand concentration. Typical extensions to the Langmuir model are the mass transport, the heterogeneous ligand, the bivalent, the conformational change, and the heterogeneous analyte models.

The aim is to calculate kinetic parameters of a reaction network model so as to best explain the observed signal $R_{observed}(t)$ with the model signal $R_{model}(t,\vec{p})$, which in state-of-the-art applications is achieved by using the discrepancy

function

$$f_{discrepancy}(t, \vec{p}) = R_{observed}(t) - R_{model}(t, \vec{p}). \qquad (8.)$$

The model signal $R_{model}(t,\vec{p})$ depends on the parameters $\vec{p}$ that comprise the kinetic parameters.

State-of-the-art applications typically use for objective function the chi-squared expression

$$\chi^2 = \sum_t \left( f_{discrepancy}(t, \vec{p}) \right)^2, \qquad (9.)$$

which summarizes the discrepancies over time into a single scalar value obtained by summing over time the square of the discrepancy function values.

The model signal $R_{model(t,\vec{p})}$ of the Langmuir model during association is given by Equation (6.), and during dissociation is given by Equation (7.). The parameters of the Langmuir reaction network are $\vec{p} = (k_a, k_d, R_{max})$, where $k_a$ and $k_d$ are the kinetic parameters.

Methods used in state-of-the-art applications to minimize the objective function are of iterative type, including gradient based methods such as the Gauss-Newton algorithm, the Levenberg-Marquardt algorithm, and variants or extensions thereof.

The major drawback of iterative minimization methods, in particular when the model requires numerical integration, is the significant computation time required to achieve convergence. The computation time introduces significant delays in the experimenters' workflow who is unable to quickly evaluate the kinetic parameters for different configurations.

Further on, the computational complexity of iterative methods prevents real time Monte-Carlo simulations to compute the estimation error, tune hyperparameters such as the fit range, or perform model selection.

An alternative to iterative methods for determining the kinetic parameters are direct estimation methods that minimize an objective function summarizing the discrepancy of an observed signal with respect to a different discrepancy function. The discrepancy function used in direct estimation methods is defined by the differential equations of the reaction network. Direct estimation methods are methods that obtain the desired result in a single closed form computation and do not require iterative steps that converge to the solution without reaching it exactly. A common direct estimation method is ordinary least square estimation to determine the variables (i.e. desired result) for a linear system of equations.

The direct estimation method derived by Kovacs & Toth (2007) is given by

$$\hat{k} = (X^T X)^{-1} X^T \left( x(t_n) - x_0 - \int_0^{t_n} F_r\big(x(t)\big) dt \right), \qquad X = \int_0^{t_n} F\big(x(t)\big) dt, \ (10.)$$

for the reaction network equation

$$\dot{x}(t) = F\big(x(t)\big) \cdot k + F_r\big(x(t)\big), \qquad x(0) = x_0, \qquad (11.)$$

wherein the variable $x(t)$ is a vector, and the differential equation implicitly defines multiple differential equations written in vector form.

The Equations (3.a) and (3.b) are a special case of the reaction network Equation (11.), which becomes apparent when writing Equations (3.) in the form

$$\begin{pmatrix} [\dot{AB}] \\ [\dot{B}] \end{pmatrix} = \begin{pmatrix} [A][B] & -[AB] \\ -[A][B] & [AB] \end{pmatrix} \begin{pmatrix} k_a \\ k_d \end{pmatrix}, \qquad (12.)$$

where

$$x(t) = \begin{pmatrix} [AB](t) \\ [B](t) \end{pmatrix}, \qquad F\begin{pmatrix} [AB] \\ [B] \end{pmatrix} = \begin{pmatrix} [A][B] & -[AB] \\ -[A][B] & [AB] \end{pmatrix} \ \text{and} \ k = \begin{pmatrix} k_a \\ k_d \end{pmatrix}. (13.)$$

The kinetic parameters $k_a$ and $k_d$ can then be determined directly by computing the direct estimator in Equation (10.), estimator which does not require any iteration to converge to the desired result.

When the model signal equals the observed signal, and the observed signal is sampled at a time resolution smaller than the rate of change, the difference between the kinetic parameters determined with an iterative method and a direct estimation are negligible for practical purposes. When discrepancies between the observed signal and the model signal arise, significant differences can occur between the kinetic parameters determined with an iterative method and a direct estimation method. The differences depend on the statistical properties of the discrepancy values, and these differences are not mathematically understood in the general case.

The requirement of Equation (10.) to observe simultaneously the product concentration [AB] and the ligand concentration [B] rules out this direct estimation method as an alternative to determine kinetic parameters from observed signals where only the product concentration is observed, and the ligand concentration is a hidden state of the reaction network.

A solution to the limitation of direct estimation methods to signals observing all states in a reaction network has recently been proposed (Dattner, 2015). The solution involves determining an adequate functional basis to describe the observed states and hidden states. For example, in Equations (12.) and (13.) the hidden ligand state can be approximated by

$$[B](t) = \sum_{k=0}^{K} \beta_k \varphi_k(t), \qquad (14.)$$

where the $\{\varphi_0(t) \dots , \varphi_K(t)\}$ form a finite functional basis that allows for a good approximation of the ligand concentration over time (e.g. polynomial basis $\{1, x, x^2, \dots\}$), and $\beta_k$ are parameters to be determined. In vector form, any state can be written as

$$\text{state}(t) = \vec{\beta}_{state} \cdot \vec{\varphi}(t), \qquad (15.)$$

where $\vec{\beta}_{state} = \{\beta_0^{state}, \dots, \beta_K^{state}\}$ is the parameter vector and $\overline{\varphi}(t) = \{\varphi_0(t), \dots, \varphi_K(t)\}$ is the finite functional basis vector.

For observed states, the parameters $\vec{\beta}_{state}$ can be determined by a regularized linear regression estimator

$$\vec{\beta}_{state} = \left[ \left( \vec{\varphi}(\vec{t})\vec{\varphi}(\vec{t})^T \right)^{-1} \vec{\varphi}(\vec{t}) + \lambda \cdot I \right] \cdot \text{state}(\vec{t}), \qquad (16.)$$

wherein $\lambda$ is a regularization constant; the value of $\lambda$ is pre-defined by a user; $\vec{t}$ is the vector of times at which the observed states are observed and $\vec{\varphi}(\vec{t})$ is the matrix with columns given by finite functional basis vectors at the times $\vec{t}$, $\vec{\varphi}(\vec{t})^T$ is the transposed matrix of $\vec{\varphi}(\vec{t})$ and $(\vec{\varphi}(\vec{t})\vec{\varphi}(\vec{t}))^{T})^{-1}$ is the matrix inverse of the matrix product $\vec{\varphi}(\vec{t})\vec{\varphi}(\vec{t})^T$.

For the example, the kinetic parameters can then be determined as a function of the parameters $\vec{\beta}_B$ and $\vec{\beta}_{AB}$, which relate to the states as $[B](t) = \vec{\beta}_B \cdot \vec{\varphi}(t)$ and $[AB](t) = \vec{\beta}_{AB} \cdot \vec{\varphi}(t)$, using the direct estimation method of Equation (10.) as

$$\dot{X} = \sum_t \begin{pmatrix} \vec{\beta}_{AB}^T \vec{\dot{\varphi}}(t) \\ \vec{\beta}_B^T \vec{\dot{\varphi}}(t) \end{pmatrix}, X = \sum_t \begin{pmatrix} [A]\vec{\beta}_B^T \vec{\varphi}(t) & -\vec{\beta}_{AB}^T \vec{\varphi}(t) \\ -[A]\vec{\beta}_B^T \vec{\varphi}(t) & \vec{\beta}_{AB}^T \vec{\varphi}(t) \end{pmatrix} \Rightarrow \begin{pmatrix} k_a(\vec{\beta}_B, \vec{\beta}_{AB}) \\ k_d(\vec{\beta}_B, \vec{\beta}_{AB}) \end{pmatrix} = X^{-1} \dot{X}. \quad (17.)$$

The parameters $\vec{\beta}_{AB}$ are determined from the observed state $[AB](t)$ using Equation (16.). The parameters $\vec{\beta}_B$ cannot be determined because the corresponding state $[B](t)$ is a hidden state. However, initial values for $\vec{\beta}_B$ must be provided for the subsequent minimization of the objective function in Equation (18.). Initial values for $[B](t)$ are obtained from the relation $[B](t) = [B](0) - [AB](t)$, wherein $[B](0)$ is an initial ligand concentration and wherein a value for $[B](0)$ is estimated based on the maximum value over time of the observed state $[AB](t)$. The initial values of the parameters $\vec{\beta}_B$ are determined by applying Equation (16.) to the initial values for the state $[B](t) = [B](0) - [AB](t)$ wherein $[B](0)$ is replaced by the estimated value.

Solving for the kinetic parameters then becomes a minimization problem with respect to the parameter matrix

$\beta_{state}$, where the matrix element $\beta_{state}^{ij}$ corresponds to the weight of the jth basis function for the $i$th state. The parameter matrix can be written in the bi-partite form $\beta_{state} = [\beta_{hidden}, \beta_{observed}]$ where the hidden and observed states are made explicit.

For the example, the parameter matrix is given by $\beta_{state} = [\vec{\beta}_B, \vec{\beta}_{AB}]$.

The objective function of the minimization problem reads

$$argmin_{\beta_{state}} \lambda_{diff} \int \left( \beta_{state} \vec{\dot{\varphi}}(t) - F\left(\beta_{state} \cdot \vec{\varphi}(t)\right) \cdot k(\beta_{state}) - F_r\left(\beta_{state} \cdot \vec{\varphi}(t)\right) \right)^2 dt$$
$$+ \sum_{n=1}^{N} \left( \beta_{observed} \vec{\varphi}(t) - x_{observed}^{t_n} \right)^2, \qquad (18.)$$

where the first term enforces a solution to the reaction network differential Equation (11.) and the second term minimizes the chi-squared between the solution and the observed states. The parameter $\lambda_{diff}$ controls the relative weighting of the differential equation discrepancy and the discrepancy between the solution and the observed state. This parameter is initialized with $\lambda_{diff} = 0.1$ and increased each time a solution to $\beta_{state}$ is found, until the value of $\beta_{state}$ no longer changes when increasing $\lambda_{diff}$.

This minimization problem has the advantage of being dominantly linear in the parameters $\beta_{state}$ and not to require integration of the differential equations, which is computationally much more efficient to solve. However, a major drawback of the method is that it remains iterative and the determined kinetic parameters are only an approximation whose precision depends on how well the functional basis approximates the true solution of the differential equation.

Besides the limitation of direct estimation methods to observed signals that observe all states in a reaction network, the statistical properties of current direct estimation methods are not always optimal. An undesirable statistical feature that can arise in iterative methods and direct estimation methods are biases in the determined kinetic parameters. A bias arises when the expected value of a result differs from the true underlying quantitative value being estimated. Biases are difficult to correct because they require knowledge of the distribution underlying discrepancies between the observed signal and the model signal. For Gaussian independent noise in the observed signal, direct estimation methods are known to be more prone to biases and have lower statistical efficiency than iterative methods. The statistical efficiency being the number of observations needed to achieve a given performance in computing the kinetic parameters.

However, recent mathematical progress has led to the development of a bias corrected direct estimation method (Holte, 2016), robust to Gaussian time independent noise in the observed signal. The asymptotic consistency of such corrected direct estimation methods has been proven for independent observation errors (Dattner & Gugushvili, 2015; Vujacic, Dattner, Gonzalez, & Wit, 2015). Nonetheless, the statistical properties of direct estimation methods remain unknown for dependent observation errors and heavy-tailed observation errors, which are common in applications.

In summary, state-of-the-art iterative methods which are used to determine kinetic parameters of a reaction network are insufficient because they are computationally intensive and delay the workflow of evaluating observed signals. State-of-the-art direct estimation methods which are used to determine kinetic parameters of a reaction network are insufficient because they cannot be applied to observed signals that do not observe all states of a reaction network. The recent development of a direct estimation methods that applies to reaction networks with hidden states (Dattner, 2015) relies on a parametric functional approximation of the hidden states that is optimized using iterative methods; however disadvantageously this approach results only in an approximation and requires case by case educated guesses for the choice of the functional basis.

Publication "Kinetic parameter estimation for a multiresponse nonlinear reaction model" from KAMALAKANTA ROUTRAY ET AL, discloses estimating kinetic parameters for the propane oxidative dehydrogenation reaction over vanadia-alumina catalyst under steady-state conditions. A 5% V2O5/Al2O3 catalyst is synthesized using an incipient-wetness impregnation technique. Characterization studies, such as surface area measurements, Raman spectroscopy, and temperature-programmed reduction, reveal that only reducible molecularly dispersed surface vanadium oxide species are present and the Al2O3 support is not affected. Reaction data suggest that COx's (CO and CO2) are secondary products. Consequently, a single-site consecutive Mars-van Krevelen mechanism is chosen to explain the reaction data. This consecutive reaction mechanism explains the steady-state reaction data obtained as a function of reaction temperature and propane to oxygen molar ratio. The kinetic parameters are estimated using a nonlinear multiresponse analysis. A determinant criterion is used as the objective function for minimization, which is achieved with a real-coded genetic algorithm. Based on a profiling technique the kinetic parameters are tested for nonlinearity and correlation between parameters. Additional use of the profiling technique is in reparameterization and obtaining maximum likelihood intervals. The application of the estimated kinetic parameters for improved understanding of the reaction, the optimum conditions of reactor operation, and catalyst design are then discussed.

It is an aim of the present invention to obviate, or mitigate, at least some of the above-mentioned disadvantages which are associated with existing methods for calculating kinetic parameters of a reaction network.

Brief summary of the invention

According to the invention, these aims are achieved by means of a method having the features recited in the independent claims; wherein the dependent claims recite optional features of preferred embodiments.

The method of the present invention is applied to an observed signal or sequence of observed signals of said reaction network. Advantageously, the method of the present invention can be applied to observed signals that do not observe all states of a reaction network; additionally, the method of the present invention is faster than conventional iterative methods, enabling advanced statistical analysis in real time, such as estimating errors bounds in the parameters, selecting the data range with relevant information about the kinetic parameters of the reaction network, and performing model selection.

Advantageously, the method of the present invention is used to efficiently calculate kinetic parameters of a reaction network from an observed signal or sequence of observed signals of said reaction network.

It should be understood that any definitions of terms provided in the 'Description of related art' section also apply to the present description.

Brief Description of the Drawings

Figure 1 shows baseline observed signals, association observed signals and dissociation observed signals, acquired using an SPR-based biosensor, for a product concentration in the Langmuir reaction network at different analyte concentrations and a fixed initial ligand concentration.

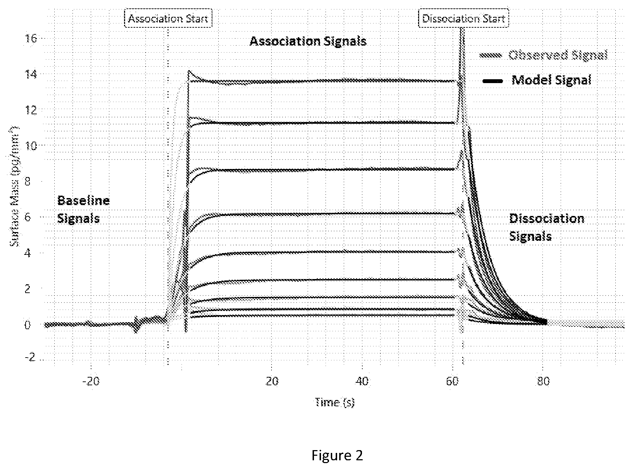

Figure 2 shows the observed signals from Figure 1 with overlaid association model signals and dissociation model signals obtained with the kinetic parameters calculated with an embodiment of the present invention.

Detailed Description of possible embodiments of the Invention

The invention will be better understood with the aid of the description of the following embodiments which are given by way of example only.

A reaction network (such as a reaction network arising in chemistry, biochemistry, pharmacokinetics, and related fields) is described by a set of differential equations; in this example the reaction network is described by a set of first order differential equations each differential equation of the set being of the form

$$\frac{d}{dt}x_i(t) = \sum_{j=1}^{R} s_{ij} \prod_{k=1}^{N} x_k^{r_{ijk}}(t), \qquad (19.)$$

where $x(t) = \{x_1(t), \dots, x_N(t)\} \in \mathbb{R}^N$ is the reaction network state, $t$ is time, $s_{ij} \in \mathbb{R}$ are the kinetic parameters of interest, and $r_{ijk}$ are stochiometric coefficients of the reaction network. The values of the stochiometric coefficients are typically determined by the law of mass action. It should be understood that different reaction networks are described by different sets of differential equations.

Typical acquisition methods output a signal that observes only some of the concentrations in the reaction network state $x(t)$ over time $t$. A state (e.g. the concentration of a component in the reaction network) in the reaction network is observed by means of a physically observable proxy state (e.g. a refractive index) which are representative of that state, such a state is an observed state; for other states, no physically observable proxy state which are representative of that state are available, such a state is a hidden state. Hereafter, to distinguish between the observed states and the hidden states in the reaction network, the reaction network state is written in the bi-part form $x(t) = \{x_o(t), x_h(t)\}$, which is the concatenation of the observed states $x_o(t)$ and the hidden states $x_h(t)$.

In the present embodiment, in order to avoid the costly computation of hidden states, the hidden states are eliminated from said set of differential equations, by substituting parameter(s) which represent hidden states with equivalent expression(s) comprising parameter(s) representing observed states, so as to form one or more intermediate differential equation(s) which are without hidden states.

To obtain said one or more intermediate differential equation(s), said set of differential equations is augmented with derivatives or integrals of said differential equations. The elimination of the hidden states may introduce higher order derivatives of the observed state into the one or more intermediate differential equations. The higher order derivatives can include second order derivatives of the observed states and/or third order derivatives of the observed states. The one or more intermediate differential equations depend only on the observed states $x_0(t)$.

The one or more intermediate differential equations can admit a vector space of solutions larger than the space of solutions to the original kinetic system of differential equations. For example, the intermediate differential equation can have solutions with negative response, which is not physically possible. To obtain the unique solution that describes the reaction network, a set of $n_i$ initial conditions $\left\{ f_{initial}^{1}\left(\vec{p}, \epsilon_1^i\right), \cdots, f_{initial}^{n_i}\left(\vec{p}, \epsilon_{n_i}^i\right)\right\}$, specific to the reaction network, are preferably enforced. Where the parameters $\left\{\epsilon_1^i, \cdots, \epsilon_{n_i}^i\right\}$ are slack variables which accommodate discrepancies of the observations from the model initial conditions. The slack variables are to be minimized.

In this example the one or more intermediate differential equations define a discrepancy function $f_{discrepancy}(t,\vec{p})$.

The discrepancy function $f_{discrepancy}(t,\vec{p})$ comprises parameters $\vec{p} = \{p_1, \dots, p_{n_1}\}$ written in vector form, said parameters $\vec{p}$ comprising the kinetic parameters of said reaction network; the parameters $\vec{p}$ of the discrepancy function may further comprise 'constants of integration' resulting from the elimination of hidden states from said set of differential equations. The expression 'constant of integration' is a terminology commonly used in the art, but herein, the 'constants of integration' are unknown parameters (i.e. they are not provided constants), which are not kinetic parameters; in this example a value for any 'constants of integration' present in the discrepancy function is determined in a subsequent step.

An objective function is then defined. The discrepancy function $f_{discrepancy}(t,\vec{p})$ is a parameter of the defined objective function. In this example, a penalized chi-squared expression (Equation 20.) defines said objective function:

$$\chi^2 = \sum_t \left( f_{discrepancy}(t, \vec{p}) \right)^2 + \vec{p}^T \Lambda \, \vec{p}, \qquad (20.)$$

wherein $f_{discrepancy}(t, \vec{p})$ is the discrepancy function, $\vec{p}$ are the parameters of the discrepancy function, and $\Lambda = \text{diag}(\lambda_1, \dots, \lambda_n)$ is a diagonal penalty matrix. $\vec{p}^T \Lambda \vec{p}$ defines an additive penalty term which penalizes each parameter $p_i$ with a respective penalty coefficient $\lambda_i$. The penalty coefficients are additional parameters. In this example, the discrepancy function $f_{discrepancy}(t,\vec{p})$ and additive penalty term $\vec{p}^T \Lambda \, \vec{p}$, define the parameters of the objective function. However, it should be understood that the additive penalty term $\vec{p}^T \Lambda \, \vec{p}$ is not essential to the invention; the objective function may have the discrepancy function $f_{discrepancy}(t,\vec{p})$ only as a parameter.

The discrepancy function $f_{discrepancy}(t,\vec{p})$ is non-linear in the parameters $\vec{p}$, which prevents the use of a direct estimation method to determine the values of the parameters that minimize the objective function. In order to determine the values for the parameters that minimize the objective function, the objective function is reparametrized so as to linearize the discrepancy function.

The discrepancy function $f_{discrepancy}(t,\vec{p})$ is linearized by reparametrizing each of its parameters $\vec{p}$ with a bijective reparameterization function $f_{\text{reparameterization}}$ that maps expressions in the parameters $\vec{p} = \{p_1, \dots, p_{n_1}\}$ to intermediate parameters $\vec{k} = \{k_1, \dots, k_{n_2}]$ as

$$\vec{k} = f_{\text{reparameterization}}(\vec{p}). \qquad (21.)$$

The number of intermediate parameters is equal to, or greater than, the number of parameters in the discrepancy function (i.e. $n_2 \geq n_1$). The inverse of the reparameterization function defines expressions in the intermediate parameters $\vec{k}$ that map to the parameters $\vec{p}$ as

$$\vec{p} = f_{\text{reparameterization}}^{-1}(\vec{k}). \qquad (22.)$$

The reparameterization of the parameters $\vec{p}$ of the discrepancy function $f_{discrepancy}(t,\vec{p})$ is done by mapping, using the inverse of the reparameterization function $f_{\text{reparameterization}}^{-1}$, each of the parameters in $\vec{p}$ in the $f_{discrepancy}(t,\vec{p})$ to a respective expression of intermediate parameter in $\vec{k}$; and then substituting each of the parameters $\vec{p}$ in the $f_{discrepancy}(t,\vec{p})$ with its respective expression in intermediate parameters in $\vec{k}$. It should be understood that discrepancy functions of different reaction networks are linearized by different reparameterization functions.

Once each of the parameters $\vec{p}$ of the discrepancy function have been substituted with their respective expression of intermediate parameters in $\vec{k}$, this yields a linearized intermediate discrepancy function that is linear in the intermediate parameter $\vec{k}$. In other words, the reparametrized discrepancy function defines a linearized intermediate discrepancy function.

In this example, the objective function further had the additive penalty term $\vec{p}^T \Lambda \vec{p}$ as a parameter. The additive penalty term $\vec{p}^T \Lambda \vec{p}$ is also reparametrized, by mapping each of the parameters in the additive penalty term to its respective expression in intermediate parameters using the inverse reparameterization function $f_{\text{reparameterization}}^{-1}$; and then substituting each of the parameters in the additive penalty term with its respective expression in intermediate parameters. As mentioned, the additive penalty term $\vec{p}^T \Lambda \vec{p}$ is not essential to the invention, accordingly the step of reparametrizing the additive penalty term $\vec{p}^T \Lambda \vec{p}$ is not essential to the invention.

Thus, as described above, the present invention involves a step of reparametrizing the objective function; specifically, if the objective function has only the discrepancy function $f_{discrepancy}(t,\vec{p})$ as a parameter then the step of reparametrizing the objective function involves reparametrizing the discrepancy function $f_{discrepancy}(t,\vec{p})$ only; if on the other hand the objective function has the discrepancy function $f_{discrepancy}(t,\vec{p})$ as a parameter and has one or more other parameters (such as the additive penalty term $\vec{p}^T \vec{p}$ mentioned in the above example) then the step of reparametrizing the objective function involves reparametrizing the discrepancy function $f_{discrepancy}(t,\vec{p})$ and reparametrizing said one or more other parameters.

Accordingly, in the above example, the objective function, now having a linearized intermediate discrepancy function (i.e. the reparametrized discrepancy function $f_{discrepancy}(t, \vec{p})$) as a parameter, and the reparametrized additive penalty term, defines an intermediate objective function.

The linearized intermediate discrepancy function is a vector with components

$$\left[ \sum_{j=1} k_j X_{ij}\big(x_o(t), \ldots, \partial_m x_o(t)\big) \right] + b_i\big(x_o(t), \ldots, \partial_m x_o(t)\big), \quad 1 \le i \le i_1 \quad (23.)$$

wherein the index i indexes the $i_1$ components of the vector, and where each component is linear in the intermediate parameters $\vec{k} = \{k_1, \ldots, k_{n_2}\}$; $X_{ij}(x_o(t), \ldots, \partial_m x_o(t))$ and $b_i(x_0(t), \ldots, \partial_m x_o(t))$ are each observation functions of observed states in the reaction network over time and derivatives of the observed state over time, wherein $m$ is the index of the highest order derivative in said intermediate differential equations. The observation functions result from the elimination of the hidden state from said set of differential equations. It should be noted that the range of the indices $i$ and $j$ in the linearized intermediate discrepancy function (i.e. intermediate differential equations) may be different from the range of the indices in the set of differential equations. The linearized intermediate differential equations that define the linearized intermediate discrepancy function consists of one or more differential equations. Each of the one or more differential equations corresponds to a respective component of the vector representing the linearized intermediate discrepancy function, said components being defined by Equation (23.).

$\{x_o(t_1), \ldots, x_o(t_T)\}$ is an observed signal, or sequence of observed signals, which represents an observed state within the reaction network at times $\{t_1, \ldots, t_T\}$; the values over time of the observation functions $X_{ij}(x_o(t), \ldots, \partial_m x_o(t))$ and $b_i(x_o(t), \ldots, \partial_m x_o(t))$ are entirely determined by values of the observed signal and the higher order derivatives of the observed signal.

In a preferred embodiment, an acquisition method is performed so as to obtain an observed signal which directly or indirectly represents an observed state, or a sequence of observed signals which directly or indirectly represent a sequence of an observed state. Preferably a label-free acquisition method is performed; and more preferably the acquisition method is performed using optical sensors with high sensitivity, large measuring range and high readout speed, particularly consisting of integrated-optical waveguides and a readout device, as they find use, for example, in pharmacology or in diagnostics.

For example, a label-free acquisition method may be performed, by attaching (immobilizing) of one or more "ligands" (a reactant such as antibodies or drug targets) to a solid support on a sensor surface (i.e. a sensitized surface on a sensor chip which is adapted to be read out by a detection scheme which outputs the measurements over time that compose the recorded observed signal). The fluid conduit containing the sensor surface is generally called flow cell or fluidic chamber and allows bringing a fluid containing the other reactant(s) to be investigated (analyte) to be brought into contact with the ligand. Thereby molecule(s) to be investigated (analyte) have the opportunity to react with the immobilized ligands at the solid support on a sensor surface and an eventual product concentration is measured and characterized. A typical molecular interaction signal is acquired by first contacting the surface-bound ligand first with a neutral buffer solution in order to establish a base signal without reaction ("baseline"), followed by contacting the surface-bound ligand with a fluid containing the actual analyte or sample (such as an antigen) so that the surface-bound ligand reacts with the analyte or sample, and the association phase where a reaction occurs can be monitored, and optionally followed by contacting the surface-bound ligand again with a neutral buffer solution in order to monitor the dissociation phase of the analyte or sample by removing the analyte from the flow cell. In other words, the concentration of the analyte is increased in a step-wise fashion for characterizing the association phase and decreased again in a step-wise fashion for characterizing the dissociation phase. Typically, the aforementioned steps are repeated for several concentrations of analyte. Typically, during the whole time of the experiment, measurements are recorded, resulting in an observed signal or sequence of observed signals, which may be further analyzed with the present invention to obtain the kinetic parameters.

An exemplary sequence of observed signals, which represent an observed state that has been obtained by performing an acquisition mention using a SPR-based biosensor, is shown in Figure 1. In this example the observed state represented by the signal is the concentration of the product resulting from an analyte reacting with a ligand. The y-axis indicates the measurement value (here in pg/mm2) and the x-axis indicates the time (here in seconds). Initially, buffer is passed over the measurement surface giving the "baseline signal". During sample injection, an increase in the observed signal is due to the reaction between the ligand and the analyte until the observed signal plateaus when the equilibrium state is reached. The measurements acquired during sample injection give the "association signal". At the end of sample injection, the sample is replaced with a continuous flow of buffer and a decrease in the observed signal reflects the dissociation, or release, of analyte from the surface. The measurements acquired during the continuous flow of buffer give the "dissociation signal". The slope of the association/dissociation curves provides valuable information regarding the kinetic parameters, and the observed signal represents surface concentration (i.e., the concentration of the product is related to the change in product density on the surface). The observed signal(s) obtained by performing an acquisition method using biosensor(s) based on other detection principles will have a similar appearance.

Typically, a reaction network is characterized by a sequence of observed signals. A typical sequence of observed signals is characterized by repeatedly acquiring a baseline signal, an association signal, and a dissociation signal for different concentrations of a same analyte. Wherein any or several of the steps of acquiring a baseline signal, an association signal, or a dissociation signal can be skipped. The lowest and highest measured analyte concentrations define an analyte concentration range. The kinetic parameters only depend on the analyte and ligand substance but do not depend on the analyte concentration. The aim is to determine the kinetic parameters values of a reaction network that best explain the entire sequence of observed signals, said observed signals being acquired at different analyte concentrations. Determining the said values of the kinetic parameters is therefore done using the entire sequence of observed signals, or a suitable sub-sequence of the observed signals. Hereafter, an observed signal is used interchangeably to designate a single observed signal or a sequence of observed signals.

In another embodiment, the observed signal or sequence of observed signals is obtained performing other acquisition methods to acquire a molecular interaction signal, such as acquisition methods with sensor(s) that operate using a marker.

In another embodiment, the observed signal or sequence of observed signals is obtained with an acquisition method used in the field of bioprocessing, which are important acquisition methods in a wide variety of industries such as pharmaceutical, genetics, food, ecology and water treatment. In bioprocessing acquisition methods (and techniques) a measurement of the reaction network state, or part of the reaction network state, relies on optical chemical sensor technologies, as for example the optical chemical sensor technology described in (US 7,041,493 B2). In such optical chemical sensor technologies, an excitation source produces light which excites an optical chemical sensor to generate emission and/or cause absorption. The emission and/or absorption is measured by a detector. The luminescence emitted from the chemical sensor or the amount of light absorbed by the chemical sensor is related to the concentration of an analyte. Such as oxygen. If the luminescence emitted changes, or if the amount of light absorbed changes, then the concentration of the analyte has changed. Further examples of measurements are carbon dioxide concentration, biomass concentration, oxygen concentration, substrate concentration, and glucose concentration.

The one or more observed signal(s) (representing observed states within the reaction network), obtained with an acquisition method, are used to form an observation matrix as follows:

For given observed signal(s) over time $\{x_o(t_1), \dots, x_o(t_T)\}$, the values of the observation functions $X_{ij}(x_o(t), \dots, \partial_m x_o(t))$ over time are summarized in an observation matrix, as shown in Equation (24.) below.

$$X = \begin{pmatrix} X_{1,1}\big(x_o(t_1),\dots,\partial_m x_o(t_1)\big) & \cdots & X_{1,n_2}\big(x_o(t_1),\dots,\partial_m x_o(t_1)\big) \\ \vdots & & \vdots \\ X_{n,1}\big(x_o(t_1),\dots,\partial_m x_o(t_1)\big) & \cdots & X_{n,n_2}\big(x_o(t_1),\dots,\partial_m x_o(t_1)\big) \\ \vdots & & \vdots \\ X_{1,1}\big(x_o(t_T),\dots,\partial_m x_o(t_T)\big) & \dots & X_{1,n_2}\big(x_o(t_T),\dots,\partial_m x_o(t_T)\big) \\ \vdots & & \vdots \end{pmatrix} \quad (24.)$$

Each column in the observation matrix corresponds to a respective intermediate parameter. The first column corresponds to the intermediate parameter $k_1$, the second column to the intermediate parameter $k_2$, and so on. The rows in the observation matrix summarize the components of the intermediate discrepancy function and the individual measurements of the observed signal. The first consecutive $i_1$ rows (where $i_1$ is the number of components in the intermediate discrepancy function) correspond to the $i_1$ components of the intermediate discrepancy function at time $t_1$, the following $i_1$ consecutive rows correspond to the $i_1$ components of the intermediate discrepancy function at time $t_2$, and so on.

It is to be understood that an equivalent observation matrix and observation vector can be obtained by: permuting the rows are equivalent; or permuting the columns of the observation matrix and the respective intermediate parameters.

Analogous to the observation matrix, the values of the observation function $b_i(x_o(t),\dots,\partial_m x_o(t))$ are summarized in an observation vector $\boldsymbol{b}$.

The observation matrix and observation vector are typically built from a sequence of observed signals. For example, reaction networks representing a molecular interaction signal have for observed state an analyte $[A]$ and a product concentration $[AB]$. The analyte concentration [A] is given a sequence of provided values (i.e. provided by the user)

$$[A]_1 \to \cdots \to [A]_i \to \cdots \to [A]_{n_3}, \qquad (25.)$$

each imposed to the reaction network for a time interval $T_i$. A positive analyte concentration interval give rise to an association observed signal and an interval with an analyte concentration of zero gives rise to a dissociation observed signal. The sequence of analyte concentrations gives rises to a respective sequence of observed signals.

To build the observation matrix and observation vector from a sequence of observed signals, the first step comprises determining for each observed signal, which are indexed by i in the sequence of observed signals, the respective interval observation matrix $\boldsymbol{X}_i$ and interval observation vector $\boldsymbol{b}_i$.

The observation matrix of a single observed signal is composed of two parts: a shared interval observation matrix $\boldsymbol{X}_{i,0}$ related to the intermediate parameters $\vec{k}_0$ shared by all observed signals in the sequence, and an interval specific observation matrix $\boldsymbol{X}_{i,1}$ related to the interval intermediate parameters $\overline{k}_{i,1}$ that are specific to that interval.

Interval specific intermediate parameters occur for example in observed signals obtained by performing a label-free acquisition method that use a refractive index as observable proxy signal. A refractive index signal is often susceptible to bulk refractive index mismatches, such as small differences in refractive index between the neutral buffer solution and the fluid containing the sample. Such mismatches can cause an observed signal offset, which is an offset constant in time by which the observed signal differs from the true underlying observed state during that time interval. Each interval defines a single observed signal. To accurately compute the kinetic parameters from such an observed signal, the observed signal offset needs to be computed jointly with the kinetic parameters. Therefore, an interval intermediate parameter that represent an observed signal offset is introduced for each interval

The observation matrix, the observation vector, and the intermediate parameters for a sequence of observed signals are given by

$$X = \big[(\boldsymbol{X}_{1,0} + \cdots + \boldsymbol{X}_{n_3,0}), \boldsymbol{X}_{1,1}, \dots, \boldsymbol{X}_{n_3,1}\big],$$
$$\boldsymbol{b} = \boldsymbol{b}_1 + \cdots + \boldsymbol{b}_{n_3}, \text{ and}$$
$$\vec{k} = \big[\vec{k}_0, k_{1,1}, \dots, k_{n_3,1}\big], \qquad (26.)$$

where $\boldsymbol{X}_{i,0}$ is the shared interval observation matrix of interval $i$, $\boldsymbol{X}_{i,1}$ is the interval specific observation matrix of interval $i$, $\boldsymbol{b}_i$ is the observation vector of interval $i$, $\vec{k}_0$ are the shared intermediate parameters and $k_{i,1}$ are the interval specific parameter(s) of interval $i$.

It should be noted that the case of determining kinetic parameters from a single observed signal is done by using a sequence of observed signals of length one, where the single observed signal is the only observed signal in the sequence.

The observation matrix and observation vector are used to form a compact representation of the components of the intermediate discrepancy function over time, as given by Equation (27.)

$$intermediate\ discrepancy\ function\ over\ time = \boldsymbol{X}\vec{k} + \boldsymbol{b} \qquad (27.)$$

$\boldsymbol{X}\vec{k} + \boldsymbol{b}$ defined a compact representation of the intermediate discrepancy function; advantageously this compact representation of the intermediate discrepancy function can be used to simplify subsequent steps.

Next, numerical values of said observation matrix and said observation vector are determined. In this example, in order to determine the numerical values of said observation matrix and said observation vector, the derivatives $\{\partial x_o(t), \dots, \partial_m x_o(t)\}$ of the observed signal which represents an observed state within the reaction network are computed. The derivatives of the observed signal can be estimated with methods known in the art, such as finite differentiation, local polynomial regression, convolutional filters, or a kernel smoother.

In one embodiment of the present invention, the derivatives of the observed signal are computed using the central finite difference scheme

$$\frac{d^n x_o}{dt^n}(t) = \sum_{i=0}^{n} (-1)^i \binom{n}{i} x_o \left( t + \left( \frac{n}{2} - i \right) \Delta t \right). \qquad (28.)$$

In a preferred embodiment, a smoothed observed signal and smoothed observed signal derivative(s) are used instead of the observed signal, respectively instead of the observed signal derivative(s). The smoothing alleviates the drawback of finite difference schemes, including the central finite difference scheme, which is their sensitivity to noise. The smoothing of the observed signal and observed signal derivative(s) can be performed by methods known to a person skilled in the art, such as local polynomial regression methods, kernel smoothing methods, smoothing spline methods, or convolutional filter methods.

In an embodiment of the present invention, the smoothed observed signal and derivatives of the smoothed observed signal are estimated using smoothing splines. The smoothing spline method estimates, in a first step, a smoothed observed signal satisfying the equation

$$\sum_{i=1}^{n} \left( x_o(t_i) - \hat{f}(t_i) \right)^2 + \lambda \int \hat{f}''(t) dt, \qquad (29.)$$

where $\hat{f}(t_i)$ is the value of the smoothed observed signal at time $t_i$, and $\lambda$ is a penalty term controlling the smoothness. The values of the smoothed signal can be computed from the observed signals in closed form using methods known in the art. In a second step, the interpolating splines are then computed from the smoothed observed signal. The derivatives of the smoothed observed signal are then estimated at a given time by computing the derivatives of the interpolating spline at that time. In a subsequent direct estimation, the smoothed observed signal is used in place of the observed signal and the smoothed derivative estimates are used in place of the derivative estimates obtained in Equation (28.).

Next the compact representation of the linearized intermediate discrepancy function $\boldsymbol{X}\vec{k} + \boldsymbol{b}$ is substituted for the linearized intermediate discrepancy function which is a parameter of the intermediate objective function, so that the intermediate objective function becomes a compact intermediate objective function:

$$\chi^2_{intermediate} = \left( \boldsymbol{X}\vec{k} + \boldsymbol{b} \right)^2 + \vec{k}^T \Lambda' \vec{k}, \qquad (30.)$$

wherein $\boldsymbol{X}$ is the observation matrix, $\boldsymbol{b}$ is the observation vector, $\vec{k}$ are the intermediate parameters in vector form of the linearized intermediate discrepancy function, and $\Lambda'$ is a diagonal matrix of intermediate additional parameters that penalize the intermediate parameters $\vec{k}$ of the linearized intermediate discrepancy function. Equation (30.) defines a compact intermediate objective function.

The minimum with respect to the intermediate parameters of the compact intermediate objective function defined in Equation (30.) is found by setting the gradient of the compact intermediate objective function with respect to the intermediate parameters to zero, as given by the equation

$$\frac{d\chi^2_{intermediate}}{d\vec{k}} = (\boldsymbol{X}^T\boldsymbol{X} + \Lambda')\vec{k} + \boldsymbol{X}^T\boldsymbol{b} = 0, \qquad (31.)$$

wherein $\boldsymbol{X}$ is the observation matrix, $\boldsymbol{b}$ is observation vector, A' is the diagonal matrix of intermediate additional penalty parameters, and $\vec{k}$ are the intermediate parameters.

The values of the intermediate parameters $\vec{k}$ that satisfy Equation (31.) are given by the direct estimation method

$$\vec{k} = -(\boldsymbol{X}^T\boldsymbol{X} + \Lambda')^{-1}\boldsymbol{X}^T\boldsymbol{b}, \qquad (32.)$$

which are the values that minimize the compact intermediate objective function. The values of the diagonal matrix of intermediate additional penalty parameters (A') in the direct estimation method of Equation (32.) are pre-defined by the user. In a simple embodiment, the user can set the diagonal matrix of intermediate additional penalty parameters to zero. In further embodiments, the user can determine the diagonal matrix of intermediate additional penalty parameters using methods known in the art, such as the Tikhonov regularization method, or the cross-validation method.

In the present example, a direct estimation method is used to determine values of the intermediate parameters $\vec{k}$ that satisfy Equation (31.); it should be understood that any suitable direct estimation method could be used. Preferably a robust least square regression estimation method is used.

The observed signal typically has a variety of quality issues as for example baseline slopes, spikes from air bubbles, oscillations and jumps originating from the physically observable proxy signal obtained by performing an acquisition method using biosensor(s) or sensor(s) used in bioprocessing. Such systematic deviations of the observed (proxy) signal from the model signal introduce strong auto-correlations and heteroscedasticity in the residuals. To mitigate the impact of quality issues in the observed signal, further embodiments determine values of the intermediate parameters $\vec{k}$ that satisfy Equation (31.) with a robust and bias corrected direct estimation method; this minimizes statistical biases and estimation errors in the estimated intermediate parameters, said biases and estimation errors depending on the statistical structure of the observed signal. In these further embodiments the direct estimation method which is used to determine values of the intermediate parameters $\vec{k}$ that satisfy Equation (31.) can be any other known direct estimation method, such as the generalized least square estimation method, the iteratively reweighted least square estimation method, least absolute deviation estimation method, Bayesian linear regression estimation method, and other variants of robust closed form estimation methods.

In a further embodiment, the parameters are constrained to a physically plausible range with a provided lower and upper bound. Hard constraints on the parameters are implemented as follows. First, the constraints on the intermediate parameters are determined by applying the reparameterization function to the bounds of the range to which the parameters are constrained. Then the constraints on the intermediate parameters are enforced by using a direct estimation method such as non-negative least square regression or bounded-variable least squares, methods known to a person skilled in the art. The enforcement of the constraints on the intermediate parameters implies that the constraints on the parameters are satisfied as well.

As mentioned, the values of the intermediate parameters $\vec{k}$ that satisfy Equation (31.) are the values that minimize the compact intermediate objective function; and these values are determined using a suitable direct estimation method. Once the values for the intermediate parameters $\vec{k}$ that minimize the compact intermediate objective function have been determined, the values of the parameters $\vec{p}$ that minimize the objective function are then determined.

The values of the parameters $\vec{p}$ that minimize the objective function are determined by computing for each parameter $\vec{p}$ the value of the respective expression in the intermediate parameters $\vec{k}$, said expression being determined from the inverse of the reparameterization function $f^{-1}_{reparameterization}$.

When the intermediate differential equation admits a vector space of solutions, an additional set of initial conditions $\left\{ f^1_{initial}(\vec{p}) = 0, \cdots, f^{n_i}_{initial}(\vec{p}) = 0 \right\}$ are preferably enforced, because the values of the parameters $\vec{p}$ cannot be uniquely determined from the inverse of the reparameterization function $f^{-1}_{reparameterization}$. Instead, the values of the parameters $\vec{p}$ are determined by the minimization problem

$$\underset{\vec{p},\vec{\epsilon}}{\mathrm{argmin}}\left[f_{\mathrm{reparameterization}}(\vec{p}) - \vec{k}\right]^2$$

$$+ \sum_{j=1}^{n_i}\left[f_{initial}^{j}(\vec{p}, \epsilon_j^i)\right]^2 + \tilde{\lambda}_j \cdot (\epsilon_j^i)^2, \qquad (33.)$$

where $\vec{p}$ are the parameters to be determined, $\vec{k}$ are the intermediate parameters with known values, $\left\{\epsilon_1^i, \cdots, \epsilon_{n_i}^i\right\}$ are slack variables to accommodate discrepancies of the observations from the model initial conditions, $\left\{f_{initial}^{1}(\vec{p}, \epsilon_1^i), \cdots, f_{initial}^{n_i}(\vec{p}, \epsilon_{n_i}^i)\right\}$ is the set of $n_i$ initial conditions for the parameters, and $\{\hat{\lambda}_1, \cdots, \hat{\lambda}_{n_i}\}$ are penalty parameters for the slack variables, and values for $\{\hat{\lambda}_1, \cdots, \hat{\lambda}_{n_i}\}$ are pre-defined by a user. The minimization can be solved using standard optimization methods such as Levenberg-Marquardt. In further embodiments, the user can determine values for the penalty parameters using methods known in the art, such as the Tikhonov regularization method, or the cross-validation method. This optimization has a very low computational cost compared to fitting the complete model to all data points.

In said step of determining the parameters $\vec{p}$ from the intermediate parameters $\vec{k}$, applying the inverse of the reparameterization function $f_{\mathrm{reparameterization}}^{-1}$ to the intermediate parameters $\vec{k}$ may introduce additional biases to the parameters $\vec{p}$. The present invention may optionally, include a step of correcting biases in the parameters $\vec{p}$; correcting biases in the parameters $\vec{p}$ may be done using standard expectation computation that relies on an assumption for the probability distribution of the intermediate parameters $\vec{k}$. The probability distribution of the intermediate parameters may be derived from the distribution of the values of the intermediate discrepancy function over time using known methods such as an analysis of variance.

Assuming that the intermediate parameters $\vec{k}$ follow a probability distribution $p(\vec{k})$, the expected value of the parameters $\vec{p}$ is given by

$$E[\vec{p}] = \int_{\vec{k}} f_{\mathrm{reparameterization}}^{-1}(\vec{k})\, p(\vec{k})d\vec{k}. \qquad (34.)$$

The bias corrected value of the parameters $\vec{p}$ is given by

$$\vec{p}_{\mathrm{corrected}} = f_{\mathrm{reparameterization}}^{-1}(\vec{k}_{estimated}) + f_{\mathrm{reparameterization}}^{-1}(E[\vec{k}]) - E[\vec{p}], \qquad (35.)$$

wherein $\vec{k}_{estimated}$ is the value, and $E[\vec{k}]$ the bias corrected value, of the intermediate parameters $\vec{k}$ and $E[\vec{p}]$ is the expected value of the parameters for the chosen distributional assumption.

In an embodiment where the observed state has discrepancies with respect to the estimated reaction network differential equation, the estimated kinetic parameters do not coincide with the kinetic parameters corresponding to the solution of the reaction network model differential equations that minimizes the chi-squared with respect to the observed state. To obtain the kinetic parameter corresponding to the least square solution of the reaction network model with respect to the observed state, the estimated parameters are preferably further corrected using known methods in the art (e.g. the method by (Dattner, 2015) that performs the objective function minimization given in Equation (18.)).

More specific embodiments of the present invention, applied to specific exemplary reaction networks, will now be described with respect to the following two examples; it should be understood that these examples are non-limiting examples:

Example 1 - Langmuir Reaction Network

Assume a reversible reaction between an analyte A and a surface-bound (immobilized) capturing molecule, or ligand, B which is not diffusion or mass transfer limited and obeys pseudo first order kinetics:

$$A + B \rightleftharpoons AB. \qquad (36.)$$

The state of this reaction network is given by

$$\{x_o(t), x_h(t)\} = ([A], [AB]) @ ([B]), \qquad (37.)$$

where the concentrations of the analyte A and the product AB are the observed state, and the concentration of the ligand B is the hidden state.

The differential equations determining the reaction network state over time are

$$[\dot{A}] = 0, \qquad (a)$$
$$[\dot{B}] = -k_a[A][B] + k_d[AB], \quad (b)$$
$$[\dot{AB}] = k_a[A][B] - k_d[AB], \quad (c) \qquad (38.)$$

where the analyte concentration [A] is a known constant.

Then the integrated form of Equation (38.b) and Equation (38.c) are used to eliminate the ligand concentration as

$$[\dot{B}] = -[\dot{AB}] \quad \Rightarrow \quad [B] = R - [AB], \qquad (39.)$$

where R is an 'integration constant', namely the initial ligand concentration.

The elimination steps yield the intermediate differential equation

$$[\dot{AB}] - k_a[A](R - [AB]) + k_d[AB] = 0, \qquad (40.)$$

entirely determined by the observed states but non-linear in the three parameters $k_a$, $k_d$ and R. This intermediate differential equation defines the discrepancy function.

The chi-squared objective function of Equation (20.), having said discrepancy function as a parameter, is

$$\chi^2 \approx \frac{1}{T} \sum_t \left[ \begin{pmatrix} [\dot{AB}]_t \\ -[A] \\ [A][AB]_t \\ [AB]_t \end{pmatrix} \cdot \begin{pmatrix} 1 \\ k_a \cdot R \\ k_a \\ k_d \end{pmatrix} \right]^2, \qquad (41.)$$

where the objective function sums with respect to time the square of the discrepancy function. The chi-squared objective function comprises the discrepancy function defined by Equation (40.) written in vector form.

The chi-squared expression is reparametrized to the intermediate objective function

$$\chi^2 \approx \frac{1}{T} \sum_t \left[ \begin{pmatrix} [\dot{AB}]_t \\ -[A] \\ [A][AB]_t \\ [AB]_t \end{pmatrix} \cdot \begin{pmatrix} 1 \\ k_1 \\ k_2 \\ k_3 \end{pmatrix} \right]^2, \qquad (42.)$$

comprising a linearized intermediate discrepancy function that is linear with respect to the intermediate parameters $\vec{k} = (k_1, k_2, k_3)$.

The intermediate parameters $\vec{k}$ are obtained with the reparameterization function $f_{\text{reparameterization}}$ defined by

$$\begin{pmatrix} k_1 \\ k_2 \\ k_3 \end{pmatrix} = f_{\text{reparameterization}} \begin{pmatrix} R \\ k_a \\ k_d \end{pmatrix} = \begin{pmatrix} k_a \cdot R \\ k_a \\ k_d \end{pmatrix}. \qquad (43.)$$

The reparameterization function has for inverse

$$\begin{pmatrix} R \\ k_a \\ k_d \end{pmatrix} = f_{\text{reparameterization}}^{-1} \begin{pmatrix} k_1 \\ k_2 \\ k_3 \end{pmatrix} = \begin{pmatrix} k_1/k_2 \\ k_2 \\ k_3 \end{pmatrix}. \qquad (44.)$$

The one or more observed signal(s) (representing observed states within the reaction network), obtained by performing an acquisition method, are used to form an observation matrix as follows:

The observation matrix for this example, as defined in Equation (24.), is given by

$$\boldsymbol{X} = \begin{pmatrix} -[A] & [A][AB]_1 & [AB]_1 \\ \vdots & \vdots & \vdots \\ -[A] & [A][AB]_T & [AB]_T \end{pmatrix}, \qquad (45.)$$

wherein $[A]$ is the analyte concentration constant in time and $[AB]_t$ is the product concentration at time $t$, which is the observed state.

The observation vector for this example is given by

$$\boldsymbol{b} = \begin{pmatrix} [\dot{AB}]_1 \\ \vdots \\ [\dot{AB}]_T \end{pmatrix}, \qquad (46.)$$

wherein $[\dot{AB}]_t$ is the derivative against time of the observed signal at time $t$.

This example comprises only intermediate parameters shared across all intervals in a sequence of observed signals and no interval specific intermediate parameters. Therefore, all interval observed matrices are defined by Equation (45.) and all interval observed vectors are defined by Equation (46.) for a sequence of observe signals. The observation matrix and observation vector for the sequence of observed signals are given by stacking all interval observation matrices, respectively stacking all interval observation vectors. In the present application "stacking" matrices or vectors, comprises concatenating vertically said matrices or vectors. The intermediate parameters are given by $\vec{k}$.

The first order derivative of the observed signal, which represents the product concentration, is estimated with the central finite difference scheme

$$[\dot{AB}]_t = \frac{[AB]_{t-\Delta t} - [AB]_{t+\Delta t}}{2 \cdot \Delta t}, \qquad (47.)$$

wherein $\Delta t$ is the time interval between two timewise consecutive measurements.

The values of the intermediate parameters $\vec{k}$, which minimize the $\chi^2$ intermediate objective function, are given by the direct estimator

$$\vec{k} = -(\boldsymbol{X}^T\boldsymbol{X})^{-1}\boldsymbol{X}^T\boldsymbol{b}, \qquad (48.)$$

wherein the intermediate parameters $\vec{k}$ are entirely determined as an expression of the observation matrix and the observation vector. The diagonal matrix of additional penalty parameters is zero.

The parameters $k_a$, $k_d$ and $R$ are then determined by applying the inverse reparameterization function $f_{\text{reparameterization}}^{-1}$ defined in Equation (44.) to the found values for the intermediate parameters $\vec{k}$.

In this example, we assume Gaussian probability distributions

$$k_1 \sim \mathcal{N}(k_a \cdot R, \sigma_1) \text{ and } k_2 \sim \mathcal{N}(k_d, \sigma_2) \qquad (49.)$$

for the intermediate parameters $k_1$ and $k_2$, where the standard deviations $\sigma_1$, and $\sigma_2$ have been obtained by an analysis of variance of the values of the discrepancy function.

Then the probability distribution of the estimated $R$ according to the first component in Equation (44.) is the Gaussian ratio distribution

$$R \sim \frac{\mathcal{N}(k_1'|k_1,\sigma_1)}{\mathcal{N}(k_2'|k_2,\sigma_2)}, \qquad\qquad (50.)$$

which has for expected value

$$E[R] = \iint_{-\infty}^{+\infty} \frac{\mathcal{N}(k_1'|k_1,\sigma_1)}{\mathcal{N}(k_2'|k_2,\sigma_2)} dk_1' dk_2' . \qquad\qquad (51.)$$

Subtracting the expected value $E[R]$ from twice the estimated value R gives the bias corrected estimate

$$R_{corrected} = \frac{k_1}{k_2} + \frac{E[k_1]}{E[k_2]} - E[R] . \qquad\qquad (52.)$$

In an embodiment, a correction step to obtain the solution to the Langmuir model that minimizes the least square with respect to the observed state can be performed using the method of (Dattner, 2015) that comprises minimizing the objective function defined in Equation (18.).

Example 2 - Langmuir Reaction Network with an Observed Signal Offset

An observed signal can contain an observed signal offset, which is an offset constant in time by which the observed signal differs from the model signal. Observed signal offsets are particular common in observed signals which have been obtained by performing an acquisition method using a label-free sensor. In this case, to reliably estimate the kinetic parameters of the reaction network, the observed signal offset needs to be jointly determined with the kinetic parameters. The following embodiment uses a direct estimation method that determines jointly the kinetic parameters and the observed signal offset.

In such an embodiment the discrepancy function is

$$[\dot{AB}] - k_a[A](R - e - [AB]) + k_d[AB] + k_d \cdot e = 0, \qquad\qquad (53.)$$

wherein $e$ is an observed signal offset to the observed signal as $[AB] \to [AB] + e$ in Equation (40.), and which is overparametrized by $R$ and $e$ that are exchangeable degrees of freedom.

The estimation of the parameters $k_a$, $k_d$, $R$ and e is performed under the assumption of minimal absolute observed signal offset $e$, assumption which resolves the overparameterization and is enforced by appropriately constraining or penalizing $e$.

The chi-squared objective function of Equation (20.), with an additional penalty term for the observed signal offset, is given by

$$\chi^2 \approx \frac{1}{T} \sum_t \left[ \begin{pmatrix} [\dot{AB}]_t \\ -[A] \\ [A][AB]_t \\ [AB]_t \\ 1 \end{pmatrix} \cdot \begin{pmatrix} 1 \\ k_a \cdot R \\ k_a \\ k_d \\ (k_a[A] + k_d) \cdot e \end{pmatrix} \right]^2 + \lambda \cdot e^2, \qquad (54.)$$

where the objective function comprises the discrepancy function in vector form and one additional penalty term comprising one additional parameter $\lambda$.

The intermediate objective function is

$$\chi^2 \approx \frac{1}{T} \sum_t \left[ \begin{pmatrix} [\dot{AB}]_t \\ -[A] \\ [A][AB]_t \\ [AB]_t \\ 1 \end{pmatrix} \cdot \begin{pmatrix} 1 \\ k_1 \\ k_2 \\ k_3 \\ k_4 \end{pmatrix} \right]^2 + \lambda_4 \cdot k_4^2 , \qquad (55.)$$

comprising a linearized intermediate discrepancy function that is linear with respect to the intermediate parameters $\vec{k} = (k_1, k_2, k_3, k_4)$ and has one additional intermediate penalty term with additional parameter $\lambda_4$.

The intermediate parameters $\vec{k}$ and the additional parameter $\lambda$ are obtained with the reparameterization function defined by

$$\begin{pmatrix} k_1 \\ k_2 \\ k_3 \\ k_4 \\ \lambda_4 \end{pmatrix} = f_{\text{reparameterization}} \begin{pmatrix} R \\ k_a \\ k_d \\ e \\ \lambda \end{pmatrix} = \begin{pmatrix} k_a \cdot R \\ k_a \\ k_d \\ (k_a[A] + k_d) \cdot e \\ (k_a[A] + k_d)^{-2} \lambda \end{pmatrix} . \qquad (56.)$$

The reparameterization has for inverse

$$\begin{pmatrix} R \\ k_a \\ k_d \\ e \\ \lambda \end{pmatrix} = f_{\text{reparameterization}}^{-1} \begin{pmatrix} k_1 \\ k_2 \\ k_3 \\ k_4 \\ \lambda_4 \end{pmatrix} = \begin{pmatrix} k_1/k_2 \\ k_2 \\ k_3 \\ (k_2[A] + k_3)^{-1} \cdot k_4 \\ (k_2[A] + k_3)^2 \cdot \lambda_4 \end{pmatrix} . \qquad (57.)$$

Typically, the analyte concentration [A] is given a sequence of provided values (i.e. provided by the user)

$$[A]_1 \to \cdots \to [A]_i \to \cdots \to [A]_{n_3}, \qquad (58.)$$

each imposed to the reaction network for a time interval $T_i$. The time interval of the $i^{th}$ interval is given by $[t_{i,1}, t_{i,T}]$. The sequence of analyte concentrations gives rise to a respective sequence of interval observed signals.

The one or more observed signal(s) (representing observed states within the reaction network), obtained by performing an acquisition method, are used to form an observation matrix as follows:

The interval observation matrix for this example, as defined in Equation (24.), is composed of the following shared interval observation matrix and interval specific observation matrix

$$\boldsymbol{X}_{i,0} = \begin{pmatrix} -[A]_i & [A]_i[AB]_{t_{i,1}} & [AB]_{t_{i,1}} \\ \vdots & \vdots & \vdots \\ -[A]_i & [A]_i[AB]_{t_{i,T_i}} & [AB]_{t_{i,T_i}} \end{pmatrix}, \text{ and } \boldsymbol{X}_{i,1} = \begin{pmatrix} 1 \\ \vdots \\ 1 \end{pmatrix} \qquad (59.)$$

wherein $[A]_i$ is the analyte concentration constant in time for the $i^{th}$ interval and $[AB]_t$ is the product concentration at time $t$, which is the observed state.

The interval observation vector for this example is given by

$$\boldsymbol{b} = \begin{pmatrix} [\dot{AB}]_1 \\ \vdots \\ [\dot{AB}]_T \end{pmatrix}, \qquad (60.)$$

wherein $[\dot{AB}]_t$ is the derivative against time of the observed signal at time $t$.

The intermediate parameters for a sequence of observed signals are given by

$$\vec{k} = \left(k_1, k_2, k_3, k_{4,1}, \dots, k_{4,i}, \dots, k_{4,n_3}\right), \qquad (61.)$$

where the intermediate parameters $(k_1, k_2, k_3)$ are shared across all observed signal intervals and $(k_{4,1}, \dots, k_{4,i}, \dots, k_{4,n_3})$ are interval specific intermediate parameters.

The observation matrix and observation vector for a sequence of observed signals are obtained using Equation (26.) from the interval observation matrices and interval observation vectors.

The first order derivative $[\dot{AB}]$ of the observed signal, which represent the product concentration, is estimated with the central finite difference scheme defined in Equation (47.).

The values of the intermediate parameters $\vec{k}$, which minimize the $\chi^2$ intermediate objective function, are given by the direct estimator

$$\vec{k} = -(X^T X + \Lambda)^{-1} X^T b, \qquad (62.)$$

wherein the intermediate parameters $\vec{k}$ are entirely determined as an expression of the observation matrix, the observation vector, and the diagonal matrix of additional penalty parameters $A = \mathrm{diag}(0,0,0,\lambda_{4,1}, \dots, \lambda_{4,i}, \dots, \lambda_{4,n_3})$. Each interval specific intermediate parameter has a respective additional penalty parameter. The value of the additional intermediate penalty parameters $(\lambda_{4,1}, \dots, \lambda_{4,i}, \dots, \lambda_{4,n_3})$ is pre-defined by the user. In one embodiment, the additional intermediate penalty parameters are all set to zero. In further embodiments, the user applies methods known in the art to determine the additional intermediate penalty parameters, methods such as cross-validation or Tikhonov regularization.

The parameters $k_a$, $k_d$, $k_t$, $R$ and $e$ are then determined by applying the inverse reparameterization function in Equation (57.) to the values of the intermediate parameters $\vec{k}$.

When the intermediate parameters are determined from a sequence of observed signals, each interval specific parameter $k_{4,i}$ determines the interval observed signal offset as $e_i = (k_2[A] + k_3)^{-1}. k4, i$.

Assuming Gaussian distribution for the intermediate parameters $\vec{k}$, the bias correction for $R$ remains unchanged from Equation (52.) in the previous example without observed signal offset. The expected value for the observed signal offset is given by

$$E[e] = \iiint_{-\infty}^{+\infty} \frac{\mathcal{N}(k_4'|k_4, \sigma_4)}{\mathcal{N}(k_2'|k_2, \sigma_2)[A] + \mathcal{N}(k_3'|k_3, \sigma_3)} \, dk_2' dk_3' dk_4', \qquad (63.)$$

wherein the intermediate parameter $k_4$ is as well assumed to have Gaussian distribution $\mathcal{N}(k_4'|k_4, \sigma_4)$ around the determined value $k_4$ with standard deviation $\sigma_4$ determined with an analysis of variance.

The bias corrected observed signal offset $e_{corrected}$ follows from Equation (35.).

In an embodiment, a correction step to obtain the solution to the model that minimizes the least square with respect to the observed state can be performed using the method of (Dattner, 2015) that comprises minimizing the objective function defined in Equation (18.).

Example 3 - Quasi-Steady State Mass Transport Reaction Network

Common observed signals a represented by reaction networks involving an analyte diffusing to a surface and then reacting with a ligand immobilized at the surface, reaction which is described by the reaction network

$$A \rightleftharpoons A_s + B \rightleftharpoons A_s B. \qquad (64.)$$

Hereafter, $[A_s]$ is the surface analyte concentration, the concentration of the analyte at the surface where the ligand is immobilized. When the diffusion of the analyte to the surface is slow, the surface analyte concentration becomes time dependent and differs from the fixed analyte concentration $[A]$, reason the surface analyte concentration needs to be modelled.

The state of this reaction network is described by

$$\{x_o(t), x_h(t)\} = ([A], [A_s B]) @ ([A_s], [B]), \qquad (65.)$$

where the concentrations of the analyte and the complex are observed states, and the concentrations of the surface analyte and ligand are hidden states.

The differential equations determining the time evolution of the reaction network state over time are

$$[\dot{A_s}] = k_t[A] - k_t[A_s] - k_a[A_s][B] + k_d[A_sB] = 0 \quad (a)$$
$$[\dot{B}] = -k_a[A_s][B] + k_d[A_sB] \qquad\qquad (b)$$
$$[\dot{A_s}B] = k_a[A_s][B] - k_d[A_sB], \qquad\qquad (c) \qquad\qquad (66.)$$

where we assume the quasi-steady state approximation $[\dot{A_s}] = 0$ to hold true during the entire duration of the observed signal.

The Equations (66.b) and (66.c) are then used to eliminate by substitution the ligand concentration as

$$[\dot{B}] = -[\dot{A_s}B] \quad \Rightarrow \quad [B] = R - [A_sB], \qquad\qquad (67.)$$

where $R$ is an 'integration constant', namely the initial ligand concentration.

The Equations (66.a) and (66.c) are used to eliminate by substitution the surface analyte concentration as

$$[A_s] = [A] - \frac{1}{k_t}[\dot{A_s}B]. \qquad\qquad (68.)$$

The two elimination steps yield the intermediate differential equation

$$[\dot{A_s}B] - k_a\left([A] - \frac{1}{k_t}[\dot{A_s}B]\right)(R - [A_sB]) + k_d[A_sB] = 0, \qquad\qquad (69.)$$

entirely determined by the concentration of the observed states. Hereafter, the intermediate differential equation defines the discrepancy function.

In this example, the chi-squared objective function of Equation (20.) is given by

$$\chi^2 \approx \frac{1}{T}\sum_t \left[\begin{pmatrix} [\dot{A_s}B]_t \\ -[A] \\ [A_sB]_t \\ [A][A_sB]_t \\ -[\dot{A_s}B]_t[A_sB]_t \end{pmatrix} \cdot \begin{pmatrix} 1 + k_a \cdot \dfrac{R}{k_t} \\ k_a \cdot R \\ k_d \\ k_a \\ \dfrac{k_a}{k_t} \end{pmatrix}\right]^2 + \lambda \cdot \left(\frac{k_a}{k_t}\right)^2, \qquad (70.)$$

and comprises as parameter the discrepancy function in vector form and one additional penalty term comprising one additional penalty parameter $\lambda$.

The chi-squared intermediate objective function is

$$\chi^2 = \frac{1}{T}\sum_t \left[\begin{pmatrix} [\dot{A_s}B]_t \\ -[A] \\ [A_sB]_t \\ [A][A_sB]_t \\ -[\dot{A_s}B]_t[A_sB]_t \end{pmatrix} \cdot \begin{pmatrix} 1 \\ k_1 \\ k_2 \\ k_3 \\ k_4 \end{pmatrix}\right]^2 + \lambda_4 \cdot k_4^2, \qquad (71.)$$

comprising the linearized intermediate discrepancy function that is linear with respect to the intermediate parameters $\vec{k} = (k_1, k_2, k_3, k_4)$ and has one additional intermediate penalty term with additional penalty parameter $\lambda_4$. The intermediate parameter $k_4$ needs to be penalized adequately. The optimal value of the addition parameter $\lambda_4$ is determined through a known regularization scheme such as Tikhonov regularization. The value of the additional parameter $\lambda_4$ depends on the

noise in the observed signal.

The intermediate parameters $\vec{k}$ are obtained with the reparameterization function defined by

$$\begin{pmatrix} k_1 \\ k_2 \\ k_3 \\ k_4 \end{pmatrix} = f_{\text{reparameterization}} \begin{pmatrix} R \\ k_a \\ k_d \\ k_t \end{pmatrix} = \left(1 + k_a \cdot \frac{R}{k_t}\right)^{-1} \begin{pmatrix} k_a \cdot R \\ k_d \\ k_a \\ k_a/k_t \end{pmatrix}. \qquad (72.)$$

The reparameterization has for inverse

$$\begin{pmatrix} R \\ k_a \\ k_d \\ k_t \end{pmatrix} = f_{\text{reparameterization}}^{-1} \begin{pmatrix} k_1 \\ k_2 \\ k_3 \\ k_4 \end{pmatrix} = \begin{pmatrix} k_1/k_3 \\ k_3^2/(k_3 - k_1 k_4)^2 \\ k_2 k_3/(k_3 - k_1 k_4)^2 \\ k_3/k_4 \end{pmatrix}. \qquad (73.)$$

The reparameterization of the additional parameter $\lambda$ in the additional penalty term is given by the identity function

$$\lambda_4 = f_{\text{reparameterization}}(\lambda) = \lambda \quad \text{and} \quad f_{\text{reparameterization}}^{-1}(\lambda_4) = \lambda. \qquad (74.)$$

The one or more observed signal(s) (representing observed states within the reaction network), obtained with an acquisition method, are used to form an observation matrix as follows:

The observation matrix as defined in Equation (24.) is given by

$$X = \begin{pmatrix} -[A]_1 & [A_sB]_1 & [A]_0[A_sB]_1 & -[\dot{A_s}B]_1[A_sB]_1 \\ \vdots & \vdots & \vdots & \vdots \\ -[A]_T & [A_sB]_T & [A]_T[A_sB]_T & -[\dot{A_s}B]_T[A_sB]_T \end{pmatrix}, \qquad (75.)$$

wherein $[A]$ is the analyte concentration constant in time and $[AB]_t$ is the product concentration at time $t$, which represents the observed state.

The observation vector for this example is given by

$$b = \begin{pmatrix} [\dot{A_s}B]_1 \\ \vdots \\ [\dot{A_s}B]_T \end{pmatrix}, \qquad (76.)$$

wherein $[\dot{A_s}B]_t$ is the derivative against time of the observed signal at time $t$.

This example comprises only intermediate parameters shared across all intervals in a sequence of observed signals and no interval specific intermediate parameters. Therefore, all interval observed matrices are defined by Equation (75.) and all interval observed vectors are defined by Equation (76.) for a sequence of observe signals. The observation matrix and observation vector for the sequence of observed signals are given by stacking all interval observation matrices, respectively stacking all interval observation vectors. The intermediate parameters are given by $\vec{k}$.

Extending the mass transport model with an interval observed signal offset for each interval observed signal in a sequence of observed signals is analogous to Example 2, which extends the Langmuir reaction network with interval observed signal offsets.

The first order derivative $[\dot{A_s}B]$ of the product concentration is estimated using the central finite difference scheme in Equation (47.).

The values of the intermediate parameters $\vec{k}$, which minimize the $\chi^2$ intermediate objective function, are given by the direct estimator

$$\vec{k} = -(\boldsymbol{X}^T\boldsymbol{X} + \Lambda)^{-1}\boldsymbol{X}^T\boldsymbol{b}, \qquad\qquad (77.)$$

wherein the intermediate parameters $\vec{k}$ are entirely determined as an expression of the observation matrix, the observation vector, and the diagonal penalty matrix A = diag(0,0,0,$\lambda_4$). The value of the additional penalty parameter $\lambda_4$ is herein provided by the experimenter.

The parameters $k_a$, $k_d$, $k_t$ and $R$ are then determined from the intermediate parameters $\vec{k}$ using the inverse reparameterization function in Equation (73.).

The bias introduced by the reparameterization on the parameters $k_a$, $k_d$, $k_t$ and $R$ is given by

$$E\left[\begin{pmatrix} k_a \\ k_d \\ R \\ k_t \end{pmatrix}\right] - \vec{p} = -\vec{p} + \int_{\vec{k}'} \begin{pmatrix} \dfrac{k_3'^2}{k_3' - k_1'k_4'} \\ \dfrac{k_2'k_3'}{k_3' - k_1'k_4'} \\ \dfrac{k_1'}{k_3'} \\ \dfrac{k_3'}{k_4'} \end{pmatrix} p(\vec{k}')d\vec{k}', \qquad (78.)$$

where the probability distribution $p(\vec{k})$ of the intermediate parameters $\vec{k}$ is determined with known methods such as an analysis of variance of the values of the discrepancy function.

In an embodiment, a correction step to obtain the solution to the model that minimizes the least square with respect to the observed state can be performed using the method of (Dattner, 2015) that comprises minimizing the objective function defined in Equation (18.).

Example 4 - Conformational Change Reaction Network

Common observed signals are typically represented by reaction networks involving an analyte reacting with a ligand immobilized at the sensor surface to form a product; said product can then undergo a conformational state change, which is a change in the shape of a macromolecule, often induced by environmental factors. The reaction of the analyte with the ligand and the conformational state change reaction are described by the reaction network

$$A + B \rightleftharpoons AB \rightleftharpoons AB^*. \qquad\qquad (79.)$$

Hereafter, [A] is the analyte concentration, [B] is the concentration of the ligand immobilized at the sensor surface, [AB] is the concentration of the product from the reaction between the analyte and the ligand, and [AB*] is the concentration of the conformationally changed state of the product [AB]. The sum [AB] + [AB*] is the observed state, and the concentrations of the ligand B, the product AB, and the product AB*, are hidden states.

The state of this reaction network is described by

$$\{x_o(t), x_h(t)\} = ([A], [AB] + [AB^*])@([B], [AB^*]), \qquad (80.)$$

where the concentrations of the analyte and the sum of the concentrations of the products are observed states, and the concentrations of the conformational state (or the product state) and ligand are hidden states.

The differential equations determining the time evolution of the reaction network state over time are

$$[\dot{B}] = -k_a[A][B] + k_d[AB] \qquad\qquad (a)$$
$$[\dot{AB}] = k_a[A][B] - k_d[AB] - k_2[AB] + k_{-2}[AB^*] \quad (b)$$
$$[\dot{AB^*}] = k_2[AB] - k_{-2}[AB^*]. \qquad\qquad (c)$$

$$(81.)$$

In a first substitution step, the concentration $[B]$ of the ligand immobilized at the sensor surface is substituted by

$$[\dot{B}] = -[\dot{AB}] - [\dot{AB^*}] \quad \text{and} \quad [B] = R - [AB] - [AB^*], \qquad (82.)$$

in the Equations (81. a, b, c), where $R$ is an 'integration constant', namely the initial ligand concentration.

In a second substitution step, the hidden product states $[AB]$ and $[AB^*]$ in the Equations (81. a, b, c) are substituted by the observed state $[C] = [AB] + [AB^*]$, and the combination of hidden states $[D] = [AB] - [AB^*]$, using $[AB] = ([C] + [D])/2$ and $[AB^*] = ([C] - [D])/2$.

Eliminating $[D]$ from the Equations (81. a, b, c), after the first and second substitution steps, yields the intermediate differential equation

$$[\ddot{C}] + ([A] \cdot k_a + k + k_d) \cdot [\dot{C}] + (k \cdot k_a + k_{-2} \cdot k_d) \cdot [C] - [A] \cdot k \cdot k_a \cdot R = 0, \qquad (83.)$$

entirely determined by the concentration of the observed state $[C]$, where the parameter $k = k_2 + k_{-2}$ has been introduced for convenience. Hereafter, the intermediate differential equation defines the discrepancy function.

The intermediate differential equation in Equation (83.) is a linear second order differential equation, which allows for a vector space $(A_1, A_2) \in \mathbb{R}^2$ of solutions of the form

$$A_0 + A_1 \cdot e^{\lambda_1 \cdot t} + A_2 \cdot e^{\lambda_2 \cdot t}, \qquad\qquad (84.)$$

where $A_0 \in \mathbb{R}$ is a constant determined by the intermediate differential equation.

In this example, a chi-squared objective function of Equation (20.) is given by

$$\chi^2 \approx \frac{1}{T} \sum_t \left[ \begin{pmatrix} [\ddot{C}]_t \\ [A][\dot{C}]_t \\ [\dot{C}]_t \\ [A][C]_t \\ [C]_t \\ -[A] \end{pmatrix} \cdot \begin{pmatrix} 1 \\ k_a \\ k + k_d \\ k \cdot k_a \\ k_{-2} \cdot k_d \\ k \cdot k_a \cdot R \end{pmatrix} \right]^2, \qquad (85.)$$

and comprises as parameter the discrepancy function in vector form.

The chi-squared intermediate objective function is

$$\chi^2 = \frac{1}{T} \sum_t \left[ \begin{pmatrix} [\ddot{C}]_t \\ [A][\dot{C}]_t \\ [\dot{C}]_t \\ [A][C]_t \\ [C]_t \\ -[A] \end{pmatrix} \cdot \begin{pmatrix} 1 \\ k_1 \\ k_2 \\ k_3 \\ k_4 \\ k_5 \end{pmatrix} \right]^2, \qquad (86.)$$

comprising the linearized intermediate discrepancy function that is linear with respect to the intermediate parameters $\vec{k} = (k_1, k_2, k_3, k_4, k_5)$.

The intermediate parameters $\vec{k}$ are obtained with the reparameterization function defined by

$$\begin{pmatrix} k_1 \\ k_2 \\ k_3 \\ k_4 \\ k_5 \end{pmatrix} = f_{\text{reparameterization}} \begin{pmatrix} R \\ k_a \\ k_d \\ k_2 \\ k_{-2} \end{pmatrix} = \begin{pmatrix} k_a \\ k_2 + k_{-2} + k_d \\ (k_2 + k_{-2}) \cdot k_a \\ k_{-2} \cdot k_d \\ (k_2 + k_{-2}) \cdot k_a \cdot R \end{pmatrix}. \qquad (87.)$$

The reparameterization has for inverse

$$\begin{pmatrix} R \\ k_a \\ k_d \\ k_2 \\ k_{-2} \end{pmatrix} = f_{\text{reparameterization}}^{-1} \begin{pmatrix} k_1 \\ k_2 \\ k_3 \\ k_4 \\ k_5 \end{pmatrix} = \begin{pmatrix} k_5/k_3 \\ k_1 \\ k_2 - k_3/k_1 \\ k_3/k_1 - k_4/(k_2 - k_3/k_1) \\ k_4/(k_2 - k_3/k_1) \end{pmatrix}. \qquad (88.)$$

The one or more observed signal(s) (representing observed states within the reaction network), obtained with an acquisition method, are used to form an observation matrix as follows:

The observation matrix as defined in Equation (24.) is given by

$$X = \begin{pmatrix} [A]_1[\dot{C}]_1 & [\dot{C}]_1 & [A]_1[C]_1 & [C]_1 & -[A]_1 \\ \vdots & \vdots & \vdots & \vdots & \vdots \\ [A]_T[\dot{C}]_T & [\dot{C}]_T & [A]_T[C]_T & [C]_T & -[A]_T \end{pmatrix}, \qquad (89.)$$

wherein $[A]_t$ is the analyte concentration constant in time for each interval and $[C]_t = [AB]_t + [AB^*]_t$ is the sum of the product concentration $[AB]_t$ and the concentration $[AB^*]_t$ of the conformationally changed state of the product, at time $t$, which represents the observed state.

The observation vector for this example is given by

$$b = \begin{pmatrix} -[\ddot{C}]_1 \\ \vdots \\ -[\ddot{C}]_T \end{pmatrix}, \qquad (90.)$$

wherein $[\ddot{C}]_t$ is the derivative against time of the observed signal at time $t$.

The Equation ... comprises only intermediate parameters shared across all intervals in a sequence of observed signals and no interval specific intermediate parameters. Therefore, all interval observed matrices are defined by Equation (89.) and all interval observed vectors are defined by Equation (90.) for a sequence of observed signals. The observation matrix and observation vector for the sequence of observed signals are given by stacking all interval observation matrices, respectively stacking all interval observation vectors. The intermediate parameters are given by $\vec{k}$.

The derivatives $[\ddot{C}]$ and $[\dot{C}]$ of the observed states are estimated using an interpolation of the data as given in Equation (29.).

The values of the intermediate parameters $\vec{k}$, which minimize the $\chi^2$ intermediate objective function, are given by a direct estimator

$$\vec{k} = -(X^T X + \Lambda)^{-1} X^T b, \qquad (91.)$$

wherein the intermediate parameters $\vec{k}$ are entirely determined as an expression of the observation matrix, and the observation vector.

Due to the vector space of solutions to the intermediate differential equation of the conformational change model, vector space of solutions given by Equation (84.), the direct estimator in Equation (91.) is degenerate, yielding a vector space of solutions for $\vec{k}$ with equal value of the objective function . Within the vector space of solutions for $\vec{k}$, there exists a unique solution that minimizes as well the additional constraints $\left\{f_{initial}^1\left(\vec{p}, \epsilon_1^i\right), \cdots, f_{initial}^{n_i}\left(\vec{p}, \epsilon_{n_i}^i\right)\right\}$ for the initial conditions of the intermediate differential equation.

A first initial condition for the differential equation is obtained from the differential Equation (81.b) at time zero, given by

$$f_{initial}^1\left(\vec{p}, \epsilon_1^i\right) = [\dot{C}](0) - [A] \cdot k_a \cdot R + \epsilon_1^i.$$

A second initial condition for the differential equation is obtained the differential Equations (81.) at equilibrium, and is given by $f_{initial}^2\left(\vec{p}, \epsilon_2^i\right) = [C](+\infty) - [A] \cdot k \cdot k_a \cdot R/(k \cdot k_a + k_{-2} \cdot k_d) + \epsilon_2^i$. This initial condition can be used when one of the intervals reached the equilibrium state.

The parameters $k_a$, $k_d$, $k_2$, $k_{-2}$ and R are then determined from the intermediate parameters $\vec{k}$ by the minimization

$$\underset{R,k_a,k_d,k_2,k_{-2}}{\mathrm{argmin}} \left[ f_{\text{reparameterization}} \begin{pmatrix} R \\ k_a \\ k_d \\ k_2 \\ k_{-2} \end{pmatrix} - \vec{k} \right]^2$$

$$+ \sum_{i=1}^{N} \left[ \begin{array}{c} [\dot{C}_i](0) - [A]_i \cdot k_a \cdot R + \epsilon_1^i \\ [C_i](T_i) + \epsilon_2^i - \dfrac{[A]_i \cdot k \cdot k_a \cdot R}{k \cdot k_a + k_{-2} \cdot k_d} \end{array} \right]^2 + \lambda_i \cdot \epsilon_i^2 , \qquad (92.)$$

which can be performed using standard optimization methods such as Levenberg-Marquardt. This optimization has a very low computational cost compared to fitting the complete model to all data points.

In an embodiment, a correction step to obtain the solution to the model that minimizes the least square with respect to the observed state can be performed using the method of (Dattner, 2015) that comprises minimizing the objective function defined in Equation (18.).

In the above-mentioned examples there are described steps in which the intermediate objective function is determined. However, in a further embodiment of the present invention the intermediate objective function is predetermined, so said above-described steps of determining the intermediate objective function are not performed. For example, there may be provided a library or memory, containing a plurality of intermediate objective functions, each for a different reaction network. In such a case the present invention may involve selecting an intermediate objective function from the library or memory.

Additionally, in a further aspect of the present invention there is provided (preferably on a tangible data carrier) a program code arranged for causing a processor to carry out the steps described above, when said processor executes said program code to determine the kinetic parameters of the reaction network.

In one embodiment the program code may be configured to receive a user's selection of an intermediate objective function from the library or memory; and then to carry out the steps described above using said selected intermediate objective function to determine the kinetic parameters of the reaction network.

**Claims**

1. A computer implemented method of calculating kinetic parameters of a reaction network from an observed signal or sequence of observed signals of said reaction network the method comprising the steps of:

   providing an intermediate objective function, wherein said intermediate objective function comprises a linearized intermediate discrepancy function which comprises intermediate parameters which have been determined by applying a reparameterization function to the parameters of a discrepancy function, wherein the intermediate discrepancy function is linear with respect to all of said intermediate parameters;
   determining values for each of the intermediate parameters in said linearized intermediate discrepancy function, which minimize the intermediate objective function, using a direct estimation method;
   determining values for the parameters of the discrepancy function by applying an inverse of the reparameterization function to said determined values of the intermediate parameters;
   determining values for the kinetic parameters from said determined values for said parameters of the discrepancy function.

2. The method according to claim 1 wherein the intermediate objective function has been obtained by, reparametrizing an objective function, which comprises at least the discrepancy function as a parameter, wherein the discrepancy function comprises the kinetic parameters of the reaction network.

3. The method according to claim 2 wherein the step of reparametrizing the objective function to obtain said intermediate objective function comprises,
   applying the reparameterization function to the parameters of the discrepancy function at least.

4. The method according to claim 3 wherein the objective function further comprises one or more additional parameters, and,

   wherein said step of reparametrizing the objective function further comprises, reparametrizing said one or more additional parameters to obtain one or more additional intermediate parameters by applying the reparameterization function to the one or more additional parameters;
   wherein said step of determining values for each of the intermediate parameters in said linearized intermediate discrepancy function, which minimize the intermediate objective function, using the direct estimation method, comprises, determining values for each of the intermediate parameters in said linearized intermediate discrepancy function and determining values for each of the one or more additional intermediate parameters, which minimize the intermediate objective function, using a direct estimation;
   further comprising the step of determining values for the one or more additional parameters by applying an inverse of the reparameterization function to said determined value for each of the one or more additional intermediate parameters; and
   wherein said step of determining values for the kinetic parameters from said determined values for said parameters of the discrepancy function comprises, determining values for the kinetic parameters from said determined values for said parameters of the discrepancy function and said determined values for said one or more additional parameters.

5. The method according to any one of claims 2-4 wherein the objective function is a function which has been obtained from one or more differential equations which define the state of the reaction network over time, where all hidden states have been removed from said one or more differential equations.

6. The method according to any one of claim 2-4 wherein the method further comprises the steps of:

   providing one or more differential equations which define the state of a reaction network over time;
   removing one or more hidden states from said one or more differential equations by substituting one or more parameters which represent hidden states with equivalent one or more expressions comprising one or more parameters representing observed states, so as to form one or more intermediate differential equations which are without hidden states, wherein said one or more intermediate differential equations which are without hidden states define said discrepancy function.

7. The method according to claim 6, wherein the step of removing one or more hidden states from the one or more differential equations comprises,

substituting at least one parameter in the one or more differential equations which represents a ligand concentration with an equivalent expression comprising parameters representing observed states.

8. The method according to claim 6 or 7, wherein the step of removing one or more hidden states from the one or more differential equations comprises,
substituting at least one parameter in the one or more differential equations which represents an analyte concentration with an equivalent expression comprising parameters representing observed states.

9. The method according to any one of claims 5-8, wherein said linearized intermediate discrepancy function comprises, at least, parameters representing observed states, the kinetic parameters of the reaction network, and one or more integration constants resulting from the step of removing one or more hidden states from said one or more differential equations.

10. The method according to any one of claims 6-9 wherein the method comprises,

enforcing an additional set of initial conditions $\left\{f_{initial}^1(\vec{p}) = 0, \cdots, f_{initial}^{n_i}(\vec{p}) = 0\right\}$ in said one or more intermediate differential equations, wherein $\vec{p}$ are parameters of said set of initial conditions which are to be determined, and
determining values of the parameters $\vec{p}$ of said set of initial conditions by solving the following minimization problem

$$\operatorname*{argmin}_{\vec{p}, \vec{\epsilon}}\left[f_{\text{reparameterization}}(\vec{p}) - \vec{k}\right]^2$$
$$+ \sum_{j=1}^{n_i}\left[f_{initial}^j(\vec{p}, \epsilon_j^i)\right]^2 + \tilde{\lambda}_j \cdot \left(\epsilon_j^i\right)^2,$$

wherein $\vec{p}$ are parameters of said set of initial conditions which are to be determined, $\vec{k}$ are the intermediate parameters with predefined values, $\left\{\epsilon_1^i, \cdots, \epsilon_{n_i}^i\right\}$ are slack variables to accommodate discrepancies of the observations from the model initial conditions, $\left\{f_{initial}^1(\vec{p}, \epsilon_1^i), \cdots, f_{initial}^{n_i}(\vec{p}, \epsilon_{n_i}^i)\right\}$ is a set of $n_j$ initial conditions for the intermediate parameters, and $\{\tilde{\lambda}_1, \cdots, \tilde{\lambda}_{n_j}\}$ are predefined penalty parameters for the slack variables.

11. The method according to claim 1 wherein the intermediate objective function further comprises one or more additional intermediate parameters.

12. The method according to claim 11 or 4 wherein said one or more additional intermediate parameters comprise one or more penalty terms constraining the intermediate parameters of the linearized intermediate discrepancy function.

13. The method according to any one of claims 11, 12 or 4 wherein said one or more additional intermediate parameters comprise at least one parameter which represents an offset in an observed state.

14. The method according to claim 13 wherein said at least one intermediate parameter represents a refractive index offset in said observed state.

15. A tangible data carrier comprising program code arranged for causing a processor to carry out the method of any one of the claims 1 to 14 when said processor executes said program code.

**Patentansprüche**

1. Ein computerimplementiertes Verfahren zum Berechnen kinetischer Parameter eines Reaktionsnetzwerks aus einem beobachteten Signal oder einer Sequenz von beobachteten Signalen des besagten Reaktionsnetzwerks, wobei das Verfahren die folgenden Schritte umfasst:

Bereitstellen einer Zwischenzielfunktion, worin die besagte Zwischenzielfunktion eine linearisierte Zwischendiskrepanzfunktion umfasst, welche Zwischenparameter umfasst, die durch Anwenden einer Reparametrisierungsfunktion auf die Parameter einer Diskrepanzfunktion bestimmt wurden, worin die Zwischendiskrepanzfunktion in Bezug auf alle besagten Zwischenparameter linear ist;

Bestimmen von Werten für jeden der Zwischenparameter in der besagten linearisierten Zwischendiskrepanzfunktion, welche die Zwischenzielfunktion minimieren, unter Verwendung einer direkten Schätzmethode;

Bestimmen von Werten für die Parameter der Diskrepanzfunktion durch Anwenden einer Umkehrfunktion der Reparametrisierungsfunktion auf die besagten bestimmten Werte der Zwischenparameter;

Bestimmen von Werten für die kinetischen Parameter aus den besagten bestimmten Werten für die besagten Parameter der Diskrepanzfunktion.

2. Verfahren gemäss Anspruch 1, worin die Zwischenzielfunktion erhalten wurde durch:
Reparametrisierung einer Zielfunktion, welche mindestens die Diskrepanzfunktion als Parameter umfasst, worin die Diskrepanzfunktion mindestens kinetische Parameter des Reaktionsnetzwerks umfasst.

3. Verfahren gemäss Anspruch 2, worin der Schritt des Reparametrisierens der Zielfunktion zum Erhalten der besagten Zwischenzielfunktion umfasst:
Anwenden der Reparametrisierungsfunktion zumindest auf die Parameter der Diskrepanzfunktion.

4. Verfahren gemäss Anspruch 3, worin die Zielfunktion zudem einen oder mehrere zusätzliche(n) Parameter umfasst, und

worin der besagte Schritt des Neuparametrisierens der Zielfunktion zudem das Neuparametrisieren des besagten einen oder der mehreren zusätzlichen Parameter umfasst, um einen oder mehrere zusätzliche Zwischenparameter durch Anwenden einer Neuparametrisierungsfunktion auf den einen oder die mehreren zusätzlichen Parameter zu erhalten; worin der besagte Schritt des Bestimmens von Werten für jeden der Zwischenparameter in der besagten linearisierten Zwischendiskrepanzfunktion, welche die Zwischenzielfunktion unter Verwendung einer direkten Schätzmethode minimiert, umfasst: Bestimmen von Werten für jeden der Zwischenparameter in der besagten linearisierten Zwischendiskrepanzfunktion und Bestimmen von Werten für jeden der einen oder mehreren zusätzlichen Zwischenparameter, welche die Zwischenzielfunktion minimieren, unter Verwendung einer direkten Schätzung;

ferner umfassend den Schritt des Bestimmens von Werten für den einen oder die mehreren zusätzlichen Parameter durch Anwenden einer Umkehrfunktion der Reparametrisierungsfunktion auf den besagten bestimmten Wert für jeden der einen oder mehreren zusätzlichen Zwischenparameter; und

worin der besagte Schritt des Bestimmens von Werten für die kinetischen Parameter aus den besagten bestimmten Werten für die besagten Parameter der Diskrepanzfunktion das Bestimmen von Werten für die kinetischen Parameter aus den besagten bestimmten Werten für die besagten Parameter der Diskrepanzfunktion und den besagten bestimmten Werten für den besagten einen oder die mehreren zusätzlichen Parameter umfasst.

5. Verfahren gemäss irgendeinem der Ansprüche 2 bis 4, worin die Zielfunktion eine Funktion ist, welche aus einer oder mehreren Differentialgleichungen gewonnen wurde, die den Zustand eines Reaktionsnetzwerks im Laufe der Zeit definieren, wobei alle verborgenen Zustände aus dieser besagten einen oder mehreren Differentialgleichungen entfernt wurden.

6. Verfahren gemäss irgendeinem der Ansprüche 2 bis 4, wobei das Verfahren zudem die folgenden Schritte umfasst:

Bereitstellung einer oder mehrerer Differentialgleichungen, welche den Zustand eines Reaktionsnetzwerks im Laufe der Zeit definieren;

Entfernen eines oder mehrerer verborgener Zustände aus den besagten einen oder mehreren Differentialgleichungen durch Ersetzen von einem oder mehreren Parametern, welche verborgene Zustände darstellen, durch einen oder mehrere äquivalente Ausdrücke, welche einen oder mehrere Parameter umfassen, die beobachtete Zustände darstellen, um so eine oder mehrere Zwischendifferentialgleichungen zu bilden, welche keine verborgenen Zustände aufweisen, wobei die besagte eine oder mehreren Zwischendifferentialgleichungen ohne verborgene Zustände die besagte Diskrepanzfunktion definieren.

29

7. Verfahren gemäss Anspruch 6, worin der Schritt des Entfernens eines oder mehrerer verborgener Zustände aus der einen oder den mehreren Differentialgleichungen umfasst:
Ersetzen von mindestens einem Parameter in der einen oder mehreren Differentialgleichungen, der eine Ligandenkonzentration darstellt, durch einen äquivalenten Ausdruck, der Parameter umfasst, welche beobachtete Zustände darstellen.

8. Verfahren gemäss Anspruch 6 oder 7, worin der Schritt des Entfernens eines oder mehrerer verborgener Zustände aus der einen oder den mehreren Differentialgleichungen umfasst:
Ersetzen von mindestens einem Parameter in der einen oder den mehreren Differentialgleichungen, der eine Analytkonzentration darstellt, durch einen äquivalenten Ausdruck, der Parameter umfasst, welche beobachtete Zustände darstellen.

9. Das Verfahren gemäss einem der Ansprüche 5 bis 8, worin die besagte linearisierte Zwischendiskrepanzfunktion mindestens Parameter umfasst, welche beobachtete Zustände, kinetische Parameter des Reaktionsnetzwerks und eine oder mehrere Integrationskonstanten darstellen, die sich aus dem Schritt des Entfernens des einen oder der mehreren verborgenen Zustände aus der besagten einen oder den mehreren Differentialgleichungen ergeben.

10. Verfahren gemäss einem der Ansprüche 6 bis 9, worin die Verfahren umfasst:

Erzwingen eines zusätzlichen Satzes von Anfangsbedingungen $\left\{f_{initial}^1(\vec{p}) = 0, \cdots, f_{initial}^{n_i}(\vec{p}) = 0\right\}$ in der besagten einen oder den mehreren Zwischendifferentialgleichungen, wobei $\vec{p}$ Parameter des besagten Satzes von zu bestimmenden Anfangsbedingungen sind, und
Bestimmen der Werte der Parameter $\vec{p}$ des besagten Satzes von Anfangsbedingungen durch Lösen des folgenden Minimierungsproblems

$$\operatorname*{argmin}_{\vec{p},\vec{\epsilon}} \left[f_{\text{reparameterization}}(\vec{p}) - \vec{k}\right]^2$$
$$+ \sum_{j=1}^{n_i} \left[f_{initial}^j(\vec{p}, \epsilon_j^i)\right]^2 + \tilde{\lambda}_j \cdot \left(\epsilon_j^i\right)^2,$$

wobei $\vec{p}$ Parameter des besagten Satzes von zu bestimmenden Anfangsbedingungen sind, $\vec{k}$ die Zwischen-parameter mit vordefinierten Werten sind, $\left\{\epsilon_1^i, \cdots, \epsilon_{n_i}^i\right\}$ Schlupfvariablen sind, um Abweichungen der Beobachtungen von den Modellanfangsbedingungen auszugleichen, $\left\{f_{initial}^1(\vec{p}, \epsilon_1^i), \cdots, f_{initial}^{n_i}(\vec{p}, \epsilon_{n_i}^i)\right\}$ ein Satz von $n_i$ Anfangsbedingungen für die Zwischenparameter ist, und $\{\lambda_{1i}, \cdots, \tilde{\lambda}_{n_i}\}$ vordefinierte Strafparameter für die Schlupfvariablen sind.

11. Verfahren gemäss Anspruch 1, worin die Zwischenzielfunktion zudem einen oder mehrere zusätzliche Zwischenparameter umfasst.

12. Verfahren gemäss Anspruch 11 oder 4, worin der besagte eine oder die mehreren zusätzlichen Zwischenparameter einen oder mehrere Strafterme umfassen, welche die Zwischenparameter der linearisierten Zwischendiskrepanzfunktion einschränken.

13. Verfahren gemäss irgendeinem der Ansprüche 11, 12 oder 4, worin der besagte eine oder die mehreren zusätzlichen Zwischenparameter mindestens einen Parameter umfassen, welcher einen Offset in einem beobachteten Zustand darstellt.

14. Verfahren gemäss Anspruch 13, worin der besagte mindestens eine Zwischenparameter einen Brechungsindex-Offset im besagten beobachteten Zustand darstellt.

15. Ein materieller Datenträger, welcher einen Programmcode umfasst, der dazu dient, einen Prozessor zu veranlassen, das Verfahren gemäss irgendeinem der Ansprüche 1 bis 14 auszuführen, wenn der besagte Prozessor den besagten Programmcode ausführt.

**Revendications**

1. Procédé mis en oeuvre par ordinateur de calcul des paramètres cinétiques d'un réseau réactionnel à partir d'un signal observé ou d'une séquence de signaux observés dudit réseau réactionnel, le procédé comprenant les étapes suivantes :

   fournir une fonction objective intermédiaire, dans lequel ladite fonction objective intermédiaire comprend une fonction de divergence intermédiaire linéarisée qui comprend des paramètres intermédiaires qui ont été déterminés en appliquant une fonction de reparamétrage aux paramètres d'une fonction de divergence, dans lequel la fonction de divergence intermédiaire est linéaire par rapport à tous les paramètres intermédiaires ;
   déterminer les valeurs de chacun des paramètres intermédiaires de ladite fonction de divergence intermédiaire linéarisée, qui minimisent la fonction objectif intermédiaire, à l'aide d'une méthode d'estimation directe ;
   déterminer les valeurs des paramètres de la fonction de divergence en appliquant l'inverse de la fonction de reparamétrage aux valeurs déterminées des paramètres intermédiaires ;
   déterminer les valeurs des paramètres cinétiques à partir des valeurs déterminées pour les paramètres de la fonction de divergence.

2. Le procédé selon la revendication 1, dans lequel la fonction objective intermédiaire a été obtenue par, reparamétrage d'une fonction objective qui comprend au moins la fonction de divergence comme paramètre, la fonction de divergence comprenant les paramètres cinétiques du réseau de réaction.

3. Le procédé selon la revendication 2, dans lequel l'étape de reparamétrage de la fonction objective pour obtenir ladite fonction objective intermédiaire comprend,
   l'application de la fonction de reparamétrage aux paramètres de la fonction de divergence au moins.

4. Le procédé selon la revendication 3, dans lequel la fonction objective comprend en outre un ou plusieurs paramètres supplémentaires, et,

   dans lequel ladite étape de reparamétrage de la fonction objective comprend en outre le reparamétrage dudit/desdits un ou plusieurs paramètres supplémentaires pour obtenir un ou plusieurs paramètres intermédiaires supplémentaires en appliquant la fonction de reparamétrage au(x) un ou plusieurs paramètres supplémentaires ;
   dans lequel l'étape consistant à déterminer les valeurs de chacun des paramètres intermédiaires de ladite fonction de divergence intermédiaire linéarisée, qui minimisent la fonction objective intermédiaire, à l'aide du procédé d'estimation directe, comprend la détermination des valeurs de chacun des paramètres intermédiaires de ladite fonction de divergence intermédiaire linéarisée et la détermination des valeurs de chacun du/des un ou plusieurs paramètres intermédiaires supplémentaires, qui minimisent la fonction objective intermédiaire, à l'aide d'une méthode d'estimation directe ;
   comprenant en outre l'étape consistant à déterminer les valeurs d'un ou de plusieurs paramètres supplémentaires en appliquant l'inverse de la fonction de reparamétrage à ladite valeur déterminée pour chacun du/des un ou plusieurs paramètres intermédiaires supplémentaires ; et
   dans lequel l'étape de détermination des valeurs des paramètres cinétiques à partir desdites valeurs déterminées pour lesdits paramètres de la fonction de divergence comprend la détermination des valeurs des paramètres cinétiques à partir desdites valeurs déterminées pour lesdits paramètres de la fonction de divergence et desdites valeurs déterminées pour ledit/lesdits un ou plusieurs paramètres supplémentaires.

5. Le procédé selon l'une des revendications 2 à 4, dans lequel la fonction objective est une fonction obtenue à partir d'une ou plusieurs équations différentielles qui définissent l'état du réseau de réaction au fil du temps, tous les états cachés ayant été supprimés de cette ou ces équations différentielles.

6. Le procédé selon l'une des revendications 2 à 4, dans lequel Le procédé comprend en outre les étapes suivantes :

   fournir une ou plusieurs équations différentielles qui définissent l'état d'un réseau de réaction au fil du temps ;
   supprimer un ou plusieurs états cachés de ladite ou desdites équations différentielles en remplaçant un ou plusieurs paramètres représentant des états cachés par une ou plusieurs expressions équivalentes comprenant un ou plusieurs paramètres représentant des états observés, de manière à former une ou plusieurs équations différentielles intermédiaires sans états cachés, dans lesquelles ladite ou lesdites équations différentielles intermédiaires sans états cachés définissent ladite fonction de divergence.

7. Le procédé selon la revendication 6, dans lequel l'étape de suppression d'un ou plusieurs états cachés de la ou des équations différentielles comprend,
la substitution d'au moins un paramètre de la ou des équations différentielles représentant une concentration de ligand par une expression équivalente comprenant des paramètres représentant des états observés.

8. Le procédé selon la revendication 6 ou 7, dans lequel l'étape d'élimination d'un ou plusieurs états cachés de l'une ou plusieurs équations différentielles comprend,
la substitution d'au moins un paramètre de la ou de plusieurs des équations différentielles représentant une concentration d'analyte par une expression équivalente comprenant des paramètres représentant des états observés.

9. Le procédé selon l'une des revendications 5 à 8, dans lequel ladite fonction de divergence intermédiaire linéarisée comprend au moins des paramètres représentant les états observés, les paramètres cinétiques du réseau de réaction et une ou plusieurs constantes d'intégration résultant de l'étape d'élimination d'un ou de plusieurs états cachés de ladite ou de plusieurs équations différentielles.

10. Le procédé selon l'une des revendications 6 à 9, dans lequel Le procédé comprend

l'application d'un ensemble supplémentaire de conditions initiales
$$\left\{ f_{initial}^1(\vec{p}) = 0, \cdots, f_{initial}^{n_i}(\vec{p}) = 0 \right\}$$ dans une ou plusieurs équations différentielles intermédiaires, dans lesquelles $\vec{p}$ sont des paramètres de cet ensemble de conditions initiales qui doivent être déterminés, et la détermination des valeurs des paramètres $\vec{p}$ de cet ensemble de conditions initiales en résolvant le problème de minimisation suivant

$$\operatorname*{argmin}_{\vec{p},\vec{\epsilon}} \left[ f_{\text{reparameterization}}(\vec{p}) - \vec{k} \right]^2$$
$$+ \sum_{j=1}^{n_i} \left[ f_{initial}^j(\vec{p}, \epsilon_j^i) \right]^2 + \tilde{\lambda}_j \cdot \left( \epsilon_j^i \right)^2,$$

où $\vec{p}$ sont les paramètres de cet ensemble de conditions initiales à déterminer, $\vec{k}$ sont les paramètres intermédiaires avec des valeurs prédéfinies, $\left\{ \epsilon_1^i, \cdots, \epsilon_{n_i}^i \right\}$ sont les variables d'ajustement pour tenir compte des écarts entre les observations et les conditions initiales du modèle, $\left\{ f_{initial}^1\left(\vec{p}, \epsilon_1^i\right), \cdots, f_{initial}^{n_i}\left(\vec{p}, \epsilon_{n_i}^i\right) \right\}$ est un ensemble de conditions initiales $n_i$ pour les paramètres intermédiaires, et $\{\tilde{\lambda}_1, \cdots, \tilde{\lambda}_{n_i}\}$ sont les paramètres de pénalité prédéfinis pour les variables d'ajustement.

11. Le procédé selon la revendication 1, dans lequel la fonction objective intermédiaire comprend en outre un ou plusieurs paramètres intermédiaires supplémentaires.

12. Le procédé selon la revendication 11 ou 4 dans lequel ledit ou lesdits plusieurs paramètres intermédiaires supplémentaires comprennent un ou plusieurs termes de pénalité contraignant les paramètres intermédiaires de la fonction de divergence intermédiaire linéarisée.

13. Le procédé selon l'une des revendications 11, 12 ou 4 dans lequel ledit ou lesdits plusieurs paramètres intermédiaires supplémentaires comprennent au moins un paramètre qui représente un décalage dans un état observé.

14. Méthode selon la revendication 13, dans lequel ledit au moins un paramètre intermédiaire représente un décalage de l'indice de réfraction dans l'état observé.

**15.** Support de données tangible comprenant un code de programme arrangé pour amener un processeur à exécuter le procédé de l'une quelconque des revendications 1 à 14 lorsque ledit processeur exécute ledit code de programme.

Figure 1

Figure 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8325347 B2

- US 7041493 B2